# EUROPEAN PATENT APPLICATION

(11) **EP 4 834 914 A1**
(43) Date of publication of application: **04.11.2026**
(21) Application number: 24911499.2
(22) Date of filing: 27.12.2024
(51) Int. Cl.: C07K 1/04, C07K 14/00, C07K 14/605, C07K 14/575, A61K 38/16, A61P 3/10, A61P 3/04, A61P 9/00

(54) **SOLID-PHASE SYNTHESIS METHOD FOR GLP-1/GIP DUAL AGONIST**

(30) Priority: 29.12.2023 CN 202311861642
(71) Applicant: Hangzhou Zhongmeihuadong Pharmaceutical Co., Ltd., GongShu District HangZhou, Zhejiang 310011 (CN)
(72) Inventor: WANG, Zhiyong, Hangzhou, Zhejiang 310011 (CN); ZHANG, Nan, Hangzhou, Zhejiang 310011 (CN); JIANG, Chunhua, Hangzhou, Zhejiang 310011 (CN); LIU, Dongzhou, Hangzhou, Zhejiang 310011 (CN)
(74) Representative: Nederlandsch Octrooibureau
(86) International application number: PCT/CN2024/143250
(87) International publication number: WO 2025/140581

(57) **Abstract**

The present invention provides a use of a dipeptide fragment in solid-phase synthesis of a polypeptide compound, and a dipeptide fragment-based solid-phase synthesis method for a polypeptide compound. The dipeptide fragment is selected from the following structures: Gly-Gly, Ile-Aib, Tyr-Aib, Thr-αMePhe and Glu-Gly.

## Description

### TECHNICAL FIELD

The present invention relates to the field of solid-phase synthesis of polypeptide compounds. In particular, the present invention provides the use of dipeptide fragments in solid-phase synthesis of a polypeptide compound, and a method for solid-phase synthesis of a polypeptide via dipeptide fragments, the dipeptide fragments are selected from the following structures: Gly-Gly, Ile-Aib, Tyr-Aib, Thr-αMePhe and Glu-Gly.

### BACKGROUND ART

The solid-phase synthesis of polypeptide compounds uses a solid-phase resin support as a synthesis platform. The amino acids are coupled to the resin support stepwise according to a predetermined sequence, and then the synthesized peptide chain is cut with a cleavage agent. After further purification, a polypeptide is obtained with high purity.

However, when a peptide with more than twenty amino acids is synthesized, the content of oligopeptide by-products, primarily characterized by the absence of residues, significantly increases and continuously accumulate. These oligopeptide by-products are very similar to the target peptide in terms of molecular weight, solubility, polarity and structure, which brings difficulties to separation and purification.

Therefore, it is necessary to continuously improve the synthesis method of long-chain polypeptide compounds so as to increase the synthesis yield and purity of the long-chain polypeptide compounds and reduce production costs.

### DISCLOSURE OF THE INVENTION

According to one aspect of the present invention, provided is a method for solid-phase synthesis of a polypeptide compound via dipeptide fragments, characterized in that the polypeptide compound is a compound of Formula AI,

H-Tyr¹-Aib²-Glu³-Gly⁴-Thr⁵-αMePhe⁶-Thr⁷-Ser⁸-Asp⁹-Tyr¹⁰-Aib¹¹-Ile¹²-Alb¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴ -Trp²⁵ -Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO: 12),

and has the following structure: 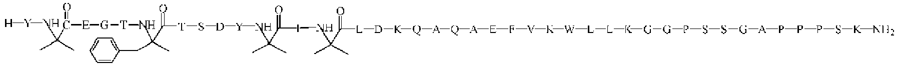
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24,
the dipeptide fragments are selected from the following structures: Gly-Gly, Ile-Aib, Tyr-Aib, Thr-αMePhe and Glu-Gly.

According to another aspect of the present invention, provided is use of dipeptide fragments in solid-phase synthesis of a polypeptide compound, characterized in that the dipeptide fragments are selected from the following structures: Gly-Gly, Ile-Aib, Tyr-Aib, Thr-αMePhe and Glu-Gly, and the polypeptide compound is a compound of Formula AI:

H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO:12),

and has the following structure: 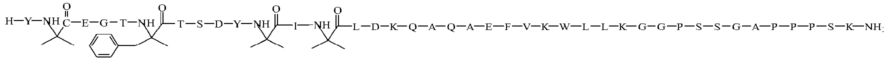
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

As compared with the solid-phase condensation coupling using single amino acids in a stepwise manner, the present invnetion uses specific dipeptide fragments for segmented coupling, avoids difficult sites that are challenging to synthesize and involve a low yield, reduces the racemization and side reactions of amino acids, and improves the synthesis yield, with a final yield up to about 10 to about 32%. Meanwhile, as for the polypeptide synthesized according to the method of the present invention, both the purity of the crude product and the purity of the pure product are improved, and the purity of the pure product can reach about 94.9 to about 99.57%. Furthermore, the method according to the present invention can be stably scaled up for production without sacrificing the final yield. For example, the method according to the present invention can stably pass through various batches of verification, including pilot batches, intermediate batches, toxicology batches, engineering batches, and GMP (Good Manufacturing Practice) batches without sacrificing the final yield and the purity of the crude product, and can also be stably scaled up from a laboratory scale of 10 mmol to a mass production scale of 50 mmol-300 mmol without sacrificing the final yield and the purity of the crude product.

As compared with the stepwise solid-phase synthesis method comprising coupling the 1^{st} to 40^{th} amino acids counting from the C-terminus in a stepwise manner, the method according to the present invention significantly improves the final yield. When compared with the large-fragment solid-phase synthesis method comprising firstly synthesizing a large fragment containing the 1^{st} to 14^{th} amino acids counting from the C-terminus, and secondly coupling the 15^{th} to 40^{th} amino acids in a stepwise manner, the large-fragment synthesis method needs a large amount of fragments (for example, 4 molar equivalents), while the method according to the present invention does not need too much excessive single amino acids and dipepitde fragments, thereby reducing production costs and improving the final yield.

### DESCRIPTION OF THE DRAWINGS

Figure 1 shows the H NMR spectrum for Compound P016.
Figure 2 shows the C NMR spectrum for Compound P016.

### DETAILED DESCRIPTION

Unless otherwise specified, all scientific and technical terms in the present invention should have the same meanings as known to those skilled in the art. If there is any inconsistency, the definitions provided in the present invention shall prevail.

It should be understood that all detailed discussions on materials, methods, examples and figures are merely for the purpose of illustration and cannot to be construed as limiting the present invention unless otherwise expressly specified.

The terms "containing" and "comprising" as used herein have the same meaning, indicating that other ingredients or other steps that do not affect the final effect may be included. Such terms include the terms "consisting of" and "substantially consisting of". The product and method according to the present invention may comprise, consist of, and substantially consist of the essential technical features and/or limitations of the present invention described herein as well as any additional and/or optional ingredients, components, steps or limitations described herein.

Unless otherwise specified, in the context of the present invention, each numerical range is meant to include both endpoints and any values and sub-ranges falling within the numerical range.

Unless otherwise specified, all materials and reagents used in the present invention are commercially available.

The following specific embodiments are applicable to both the method for solid-phase synthesis of a polypeptide compound via dipeptide fragments and the use of dipeptide fragments in solid-phase synthesis of a polypeptide compound.

### [Peptide Compounds]

According to the present invention, the polypeptide compound is a compound of Formula AI:

H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO:12),

and has the following structure: 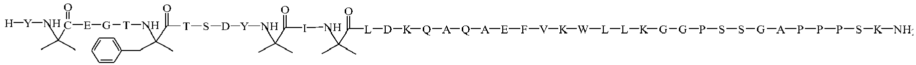
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

In some embodiments, each a is independently an integer from 1 to 5, each b is independently an integer from 1 to 5, and each c is independently an integer from 12 to 22.

The polypeptide compound of Formula AI comprises 40 amino acid residues. In the context of the present invention, unless otherwise specified or clearly contradictory, the position of an amino acid is numbered from the N-terminal on the leftmost side of the peptide chain or structural formula of the polypeptide compound. For example, taking the polypeptide compound of Formula AI as an example, the N-terminal amino acid is tyrosine (Tyr) at position 1, and the C-terminal amino acid is lysine (Lys) at position 40.

In the context of the present invention, unless otherwise specified or clearly contradictory, "polypeptide compound", "polypeptide" and "compound" are used interchangeably and all refer to the polypeptide compound of Formula AI.

In some embodiments, the polypeptide compound is a compound of Formula AI:

H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO:1),

and has the following structure: 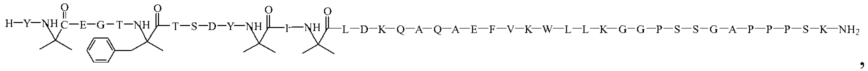 wherein at the four lysines at the position selected from position 16, position 24, position 28 and position 40, none of the side chain ε-amino group is chemically modified. That is to say, the polypeptide compound is Compound P001: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:1)

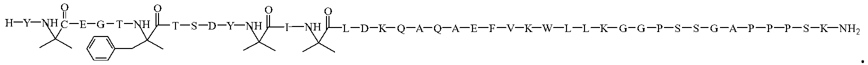

In some embodiments, the polypeptide compound is a compound of Formula AI:

H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO:12),

and
has the following structure: 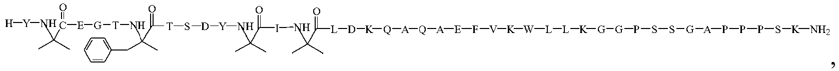
wherein, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

In a specific embodiment, the polypeptide compound of Formula AI is a compound selected from:
Compound P001: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:1) 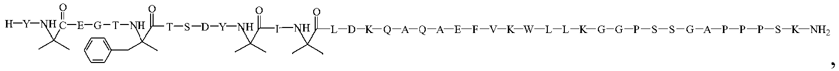
Compound P007: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-γGlu-CO-(CH₂)₁₆-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:2), having the following structure: 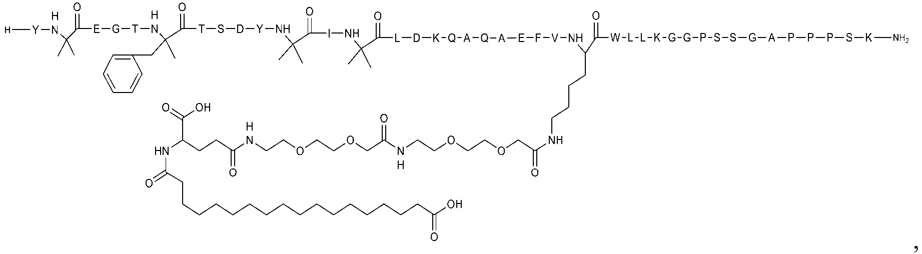
Compound P008: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴- T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-γGlu-CO-(CH₂)₁₆-COOH)-G²⁹-G³⁰-P³¹-S³²-S³³-G³⁴-A³⁵-P³⁶-P³⁷-P³⁸-S³⁹-FC⁴⁰ (SEQ ID NO:3), having the following structure: 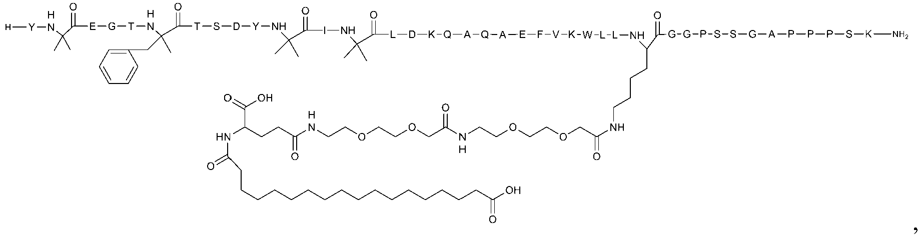
Compound P013: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²- Val²³ -Lys²⁴(AEEA-AEEA-γGlu-CO-(CH₂)₁₈-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴- T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴ (AEEA-AEEA-γGlu-CO-(CH₂)₁₈-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:4), having the following structure: 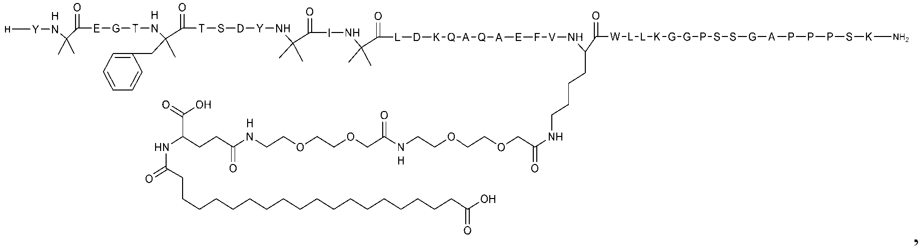
Compound P014: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:5), having the following structure: 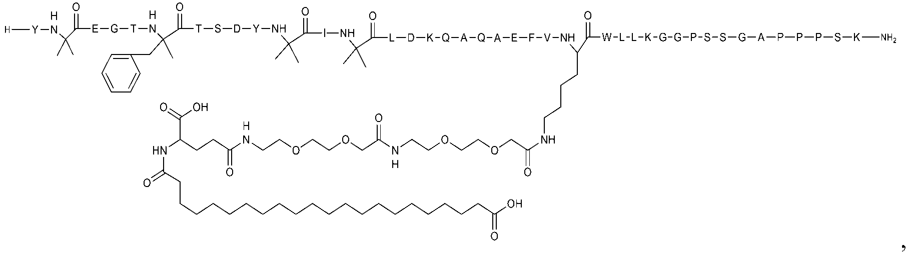
Compound P015: H-Tyr¹-Aib²-Glu³-Gly⁴-Thr^{s}-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-γGlu-yGlu-CO-(CH₂)₁₆-COOH)-Trp²⁵-Leu²⁶-Leu²⁷-Lys²⁸-Gly²⁹ -Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴ (AEEA-AEEA-γGlu-γGlu-γGlu-CO-(CH₂)₁₆-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:6), having the following structure: 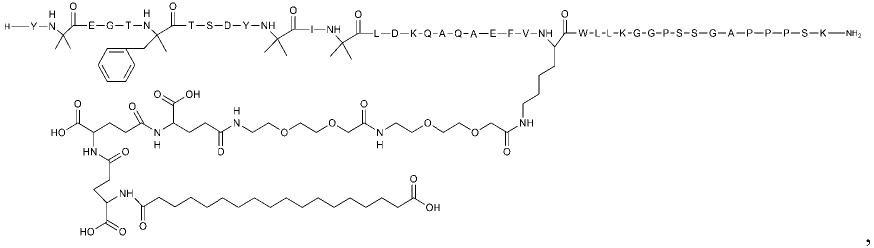
Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-FC¹⁶-Q¹⁷-A¹⁸-Q¹⁹-A²⁰-E²¹-F²²-V²³-FC²⁴(AEEA-AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:7), having the following structure: 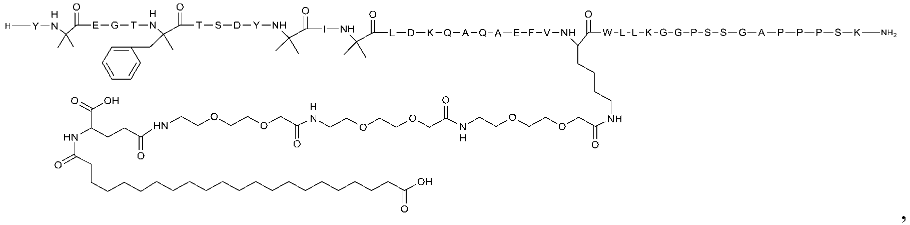
Compound P017: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹ -Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-γGlu-CO-(CH₂)₂₀-COOH)-G²⁹-G³⁰-P³¹-S³²-S³³-G³⁴-A³⁵-P³⁶-P³⁷-P³⁸-S³⁹-FC⁴⁰ (SEQ ID NO:8), having the following structure: 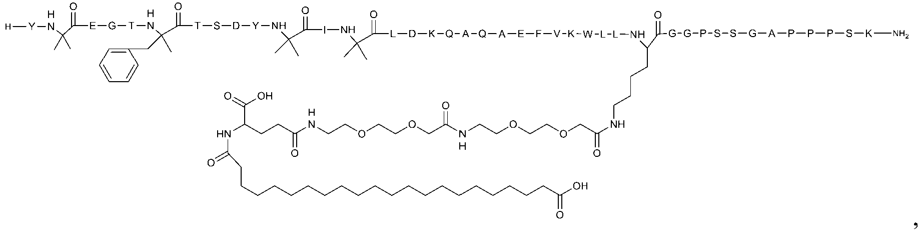
Compound P018: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-γGlu-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷ -Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-γGlu-γGlu-γGlu-CO-(CH₂)₁₆-COOH)-G²⁹-G³⁰-P³¹-S³²-S³³-G³⁴-A³⁵-P³⁶-P³⁷-P³⁸-S³⁹-K⁴⁰ (SEQ ID NO:9), having the following structure: 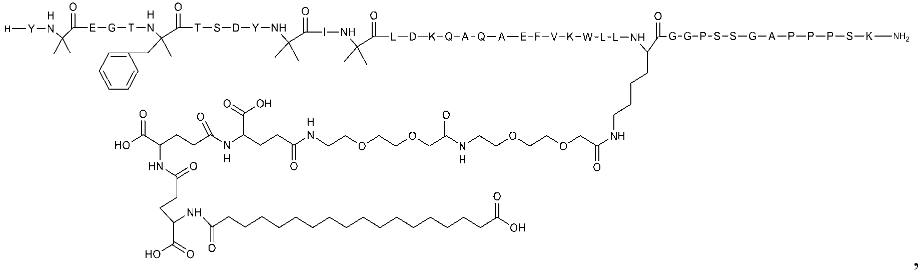
Compound P019: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-AEEA-γGlu-CO-(CH₂)₁₆-COOH)-G²⁹-G³⁰-P³¹-S³²-S³³-G³⁴-A³⁵-P³⁶-P³⁷-P³⁸-S³⁹-K⁴⁰ (SEQ ID NO:10), having the following structure: 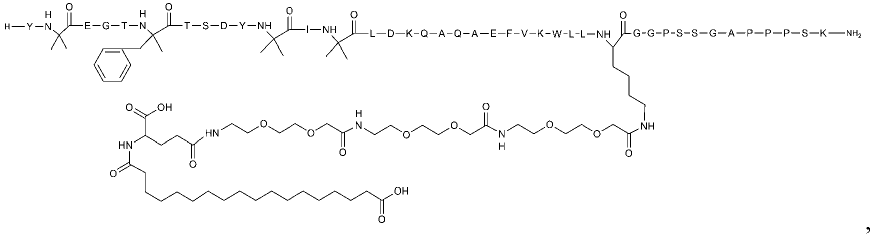 and
Compound P020: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-AEEA-γGlu-CO-(CH₂)₂₀-COOH)-G²⁹-G³⁰-P³¹-S³²-S³³-G³⁴-A³⁵-P³⁶-P³⁷-P³⁸-S³⁹-K⁴⁰ (SEQ ID NO:11), having the following structure: 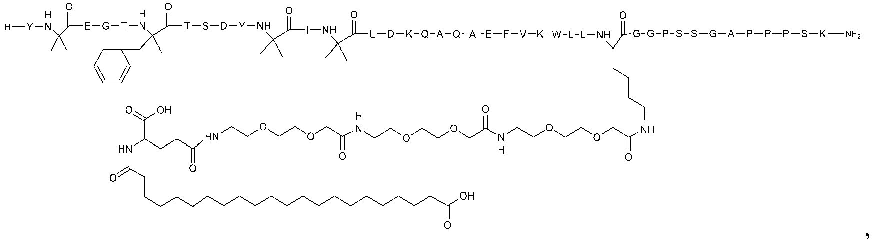
wherein AEEA represents [2-(2-amino-ethoxy)-ethoxy]-acetyl.

In a specific embodiment, the polypeptide compound is Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸. Gln¹⁹-Ala²⁰-Glu²¹-Phe²²-Val²³-Lys²⁴(AEEA -AEEA -AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, i.e., Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-FC⁴⁰, Molecular Formula being C₂₄₃H₃₈₂N₅₂O₇₃ (SEQ ID NO:7), and having the following structure:

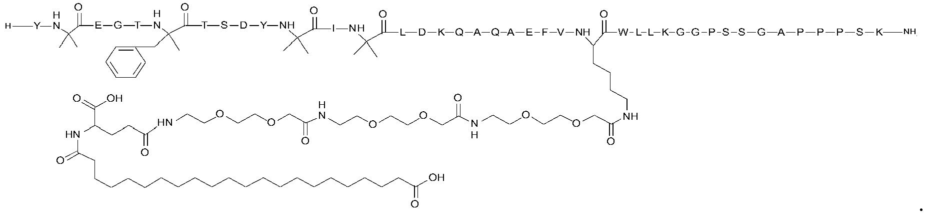

The main chain of Compound P016 comprises 40 amino acid residues, and the side chain ε-amino group of the lysine at position 24 is sequentially coupled to the following 5 moieties: AEEA, AEEA, AEEA, γ-Glu and -CO-(CH₂)₂₀-COOH.

### [Use of Dipeptide Fragments in Solid-Phase Synthesis of a Polypeptide Compound]

According to one aspect of the present invention, provided is use of dipeptide fragments in solid-phase synthesis of a polypeptide compound, characterized in that the dipeptide fragments are selected from the following structures: Gly-Gly, Ile-Aib, Tyr-Aib, Thr-αMePhe and Glu-Gly, and the polypeptide compound is a compound of Formula AI:

H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO: 12),

and has the following structure: 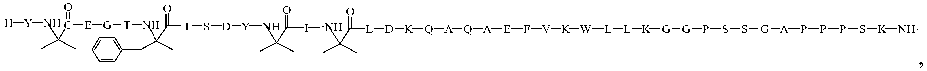
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

Unless clearly contradictory or otherwise explicitly specified, the following description to the method for solid-phase synthesis via dipeptide fragment is applicable to the use of dipeptide fragments in solid-phase synthesis of a polypeptide compound.

In some embodiments, each a is independently an integer from 1 to 5, each b is independently an integer from 1 to 5, and each c is independently an integer from 12 to 22.

### [Method for Solid-Phase Synthesis Via Dipeptide Fragments]

According to one aspect of the present invention, provided is a method for solid-phase synthesis of a polypeptide compound via dipeptide fragments, characterized in that the polypeptide compound is a compound of Formula AI,

H-Tyr¹-Aib²-Glu³-Gly⁴-Thr⁵-αMePhé-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO: 12),

and has the following structure: 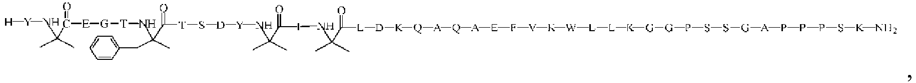
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24,
the dipeptide fragments are selected from the following structures: Gly-Gly, Ile-Aib, Tyr-Aib, Thr-αMePhe and Glu-Gly.

In the context of the present invention, " solid-phase synthesis via dipeptide fragment ", "fragment solid-phase synthesis" and "segmented coupling" are used interchangeably, all of which mean that dipeptide fragments and single amino acids are used for segmented condensation and coupling in a stepwise manner in the solid-phase synthesis method. "Condensation" and "coupling" are used interchangeably, all of which mean that the amino group of the coupling amino acid and the carboxyl of the amino acid to be coupled are dehydrated, condensated and coupled to form an amide bond or a peptide bond.

In some embodiments, each a is independently an integer from 1 to 5, each b is independently an integer from 1 to 5, and each c is independently an integer from 12 to 22.

In some embodiments, the method for solid-phase synthesis of a polypeptide compound via dipeptide fragments comprises:
preparing the peptide chain of the polypeptide compound of Formula AI by the solid-phase synthesis method via dipeptide fragments, and
optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

In some embodiments, the method for solid-phase synthesis of a polypeptide compound via dipeptide fragments comprises:
preparing the peptide chain of the polypeptide compound of Formula AI by the solid-phase synthesis method via dipeptide fragments without chemically modifying the side chain ε-amino groups of the lysines, thereby obtaining the polypeptide compound P001.

In some embodiments, the method for solid-phase synthesis of a polypeptide compound via dipeptide fragments comprises:
preparing the peptide chain of the polypeptide compound of Formula AI by the solid-phase synthesis method via dipeptide fragments, and then
at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

According to one aspect of the present invention, provided is a method for solid-phase synthesis of a polypeptide compound via dipeptide fragments, wherein the polypeptide compound of Formula AI is a compound selected from:
Compound P001: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:1) 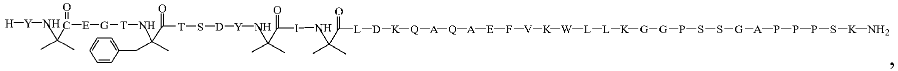
Compound P007: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴ (AEEA-AEEA-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴- T⁵-αMePhe⁶-T⁷-S⁸-D⁹ -Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-γGlu-CO-(CH₂)₁₆-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:2), having the following structure: 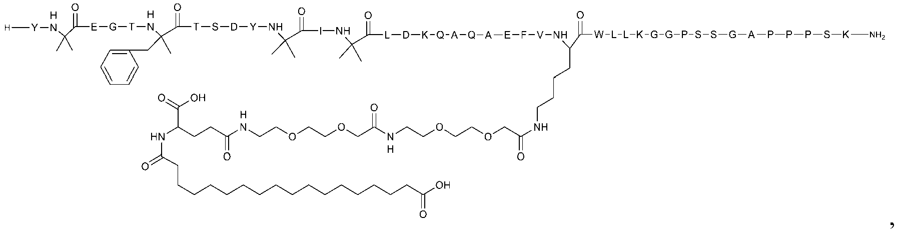
Compound P008: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸ (AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-γGlu-CO-(CH₂)₁₆-COOH)-G²⁹-G³⁰-P³¹-S³²-S³³-G³⁴-A³⁵-P³⁶-P³⁷-P³⁸-S³⁹-K⁴⁰ (SEQ ID NO:3), having the following structure: 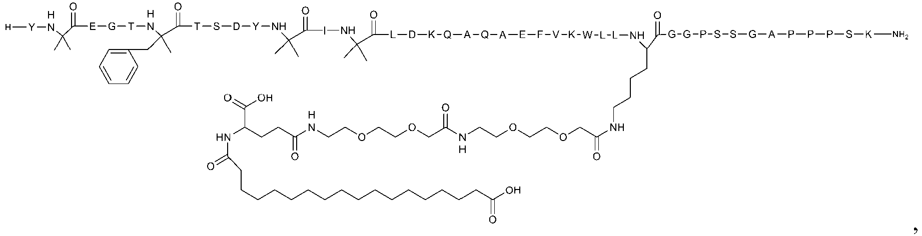
Compound P013: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₁₈-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-γGlu-CO-(CH₂)₁₈-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:4), having the following structure: 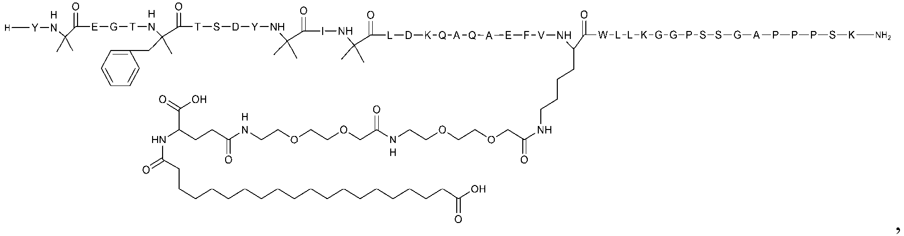
Compound P014: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²- Val²³-Lys²⁴(AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:5), having the following structure: 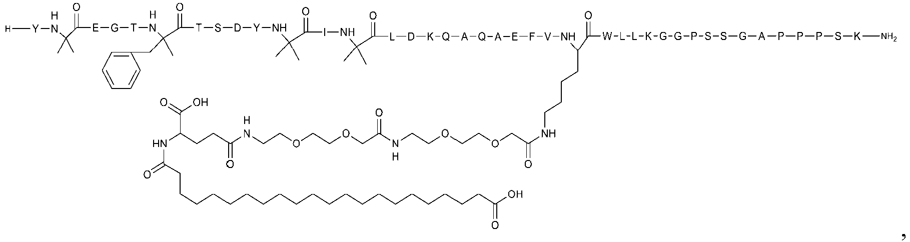
Compound P015: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-γGlu-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-γGlu-γGlu- - CO-(CH₂)₁₆-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:6), having the following structure: 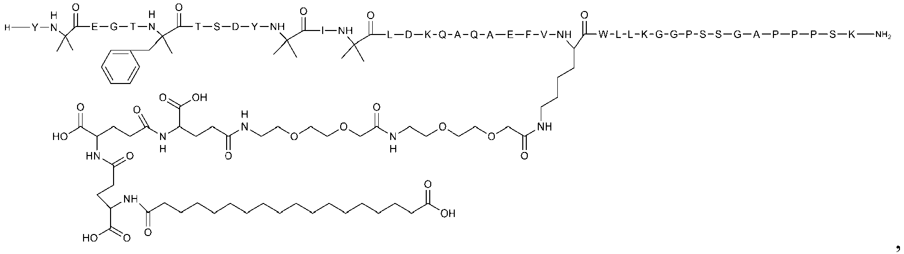
Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²- Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-FC²⁴(AEEA-AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:7), having the following structure: 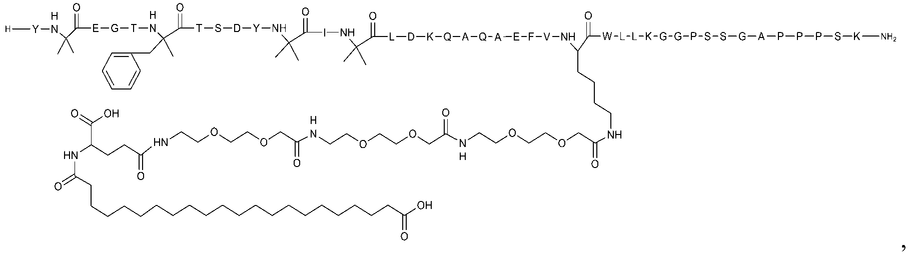
Compound P017: H-Tyr¹-Aib²-Glu³-Gly⁴-Thr^{s}-αuePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-γGlu-CO-(CH₂)₂₀-COOH)-G²⁹-G³⁰-P³¹-S³²-S³³-G³⁴-A³⁵-P³⁶-P³⁷-P³⁸-S³⁹-K⁴⁰ (SEQ ID NO:8), having the following structure: 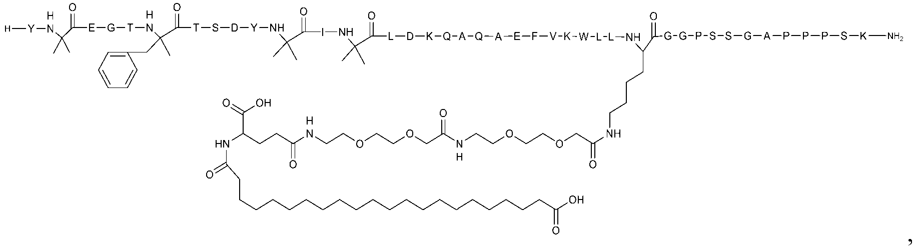
Compound P018: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-γGlu-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³ -K²⁴-W²⁵-L²⁶-L²⁷-K²⁸ (AEEA-AEEA-γGlu-γGlu-γGlu-CO-(CH₂)₁₆-COOH)-G²⁹-G³⁰-P³¹-S³²-S³³-G³⁴-A³⁵-P³⁶-P³⁷-P³⁸-S³⁹-K⁴⁰ (SEQ ID NO:9), having the following structure: 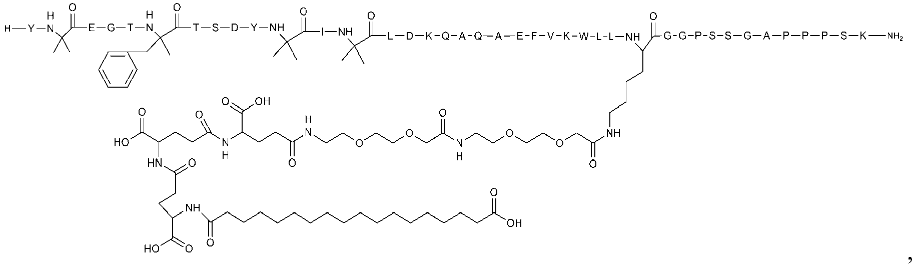
Compound P019: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸ (AEEA-AEEA-AEEA-γGlu-CO-(CH₂)₁₆-COOH)-Gly²⁹ -Gly³⁰-Pro³¹-Ser³²-Ser³³ -Gly³⁴-Ala³⁵-Pro³⁶-Pro³⁷ -Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-AEEA-γGlu-CO-(CH₂)₁₆-COOH)-G²⁹-G³⁰-P³¹-S³²-S³³-G³⁴-A³⁵-P³⁶-P³⁷-P³⁸-S³⁹ -K⁴⁰ (SEQ ID NO:10), having the following structure: 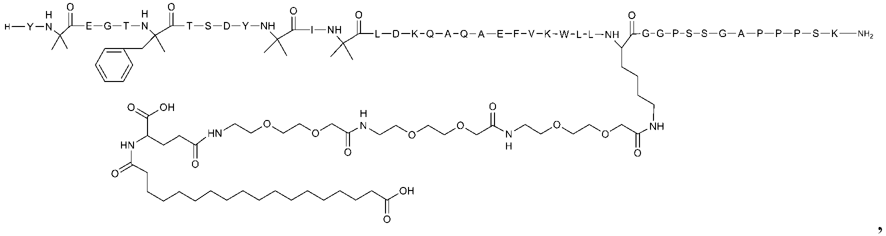 and
Compound P020: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵⁻Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷ -Pro³⁸⁻Ser³⁹-Lys⁴⁰-NH₂, namely, Y¹-Aib²-E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴-W²⁵-L²⁶-L²⁷-K²⁸(AEEA-AEEA-AEEA-γGlu-CO-(CH₂)₂₀-COOH)-G²⁹ -G³⁰-P³¹-S³²-S³³-G³⁴-A³⁵-P³⁶-P³⁷-P³⁸-S³⁹ -K⁴⁰ (SEQ ID NO:11), having the following structure: 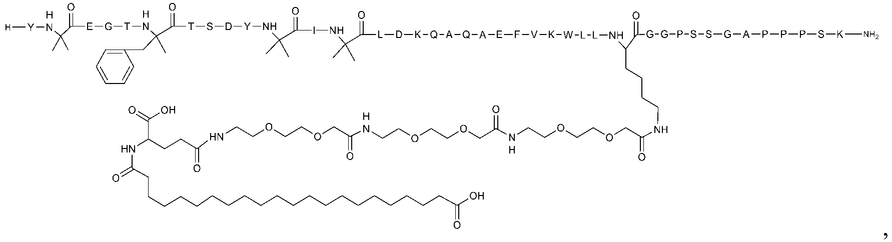
wherein AEEA represents [2-(2-amino-ethoxy)-ethoxy]-acetyl.

According to one aspect of the present invention, provided is a method for solid-phase synthesis of a polypeptide compound via dipeptide fragments, characterized in that the polypeptide compound is Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³ -Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂, i.e., Y¹-Aib² -E³-G⁴-T⁵-αMePhe⁶-T⁷-S⁸-D⁹-Y¹⁰-Aib¹¹-I¹²-Aib¹³-L¹⁴-D¹⁵-K¹⁶-Q¹⁷-A¹⁸-Q¹⁹-A²⁰-E²¹-F²²-V²³-K²⁴(AEEA-AEEA-AEEA-γGlu-CO-(CH₂)₂₀-COOH)-W²⁵-L²⁶-L²⁷-K²⁸-G²⁹-G³⁰-P³¹-S³²-S³³-G³⁴-A³⁵-P³⁶-P³⁷-P³⁸-S³⁹-K⁴⁰ (SEQ ID NO:7), molecular formula being C₂₄₃H₃₈₂N₅₂O₇₃, and having the following structure: 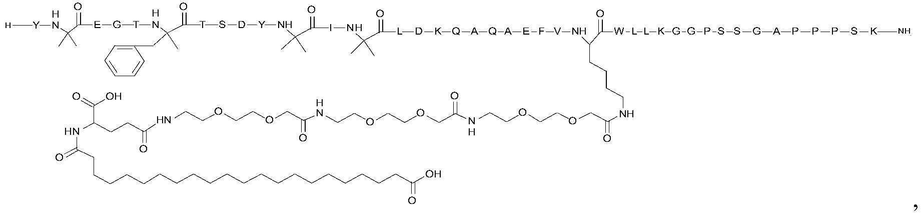 the dipeptide fragments are selected from the following structures: Gly-Gly, Ile-Aib, Tyr-Aib, Thr-αMePhe and Glu-Gly.

In some embodiments, the method for solid-phase synthesis of a polypeptide compound via dipeptide fragments comprises:
preparing the peptide chain of the polypeptide compound of Formula AI by the solid-phase synthesis method via dipeptide fragments, and then
at the lysine at position 24, chemically modifying the side chain ε-amino group with ([2-(2-amino-ethoxy)-ethoxy]-acetyl)a-(γ-Glu)b-CO-(CH₂)c-COOH to obtain the polypeptide compound P016.

### [Synthesis of Peptide Chains of Polypeptide Compounds]

Solid-phase synthesis methods commonly used for synthesizing polypeptide compounds can be used to prepare the polypeptide compound of Formula AI of the present invention. In the solid-phase synthesis method for polypeptide, single amino acids are used as building blocks to construct the polypeptide compound. The C-terminal amino acid of the peptide chain of the polypeptide compound to be coupled is protected by an amino group in advance. The carboxyl group of the amino-protected C-terminal amino acid is coupled to an insoluble resin support via a suitable linker. The amino group is then deprotected, and the C-terminal amino acid is condensed and coupled to the second amino acid (counting from the C-terminus) with activated carboxyl group and protected amino group in excess to form a peptide bond (i.e., an amide bond). Such process of condensation and coupling of amino acids is repeated sequentially until the synthesized polypeptide compound chain reaches the desired length. Next, an appropriate reagent is selected to cleave the product from the support resin, and the product is separated and purified by filtration and washing so as to obtain the peptide chain of the polypeptide compound of Formula AI.

### [Protecting Group]

In order to control the synthesis of the polypeptide compound, it is necessary to protect the functional groups on the amino acids that are not expected to undergo condensation and coupling, such as the free amino group (-NH₂) of the amino acid to be coupled, the polar groups on the side chains (such as amino (-NH₂), carboxyl (-COOH), hydroxyl (-OH)) in advance. The free amino group refers to a nitrogen atom that does not form an amide bond, and includes primary amino group (-NH₂) and secondary amino group (-NH-).

The protecting groups commonly used in solid-phase synthesis of polypeptides can be used in the present invention, as long as they do not affect the segmented coupling solid-phase synthesis method of the present invention.

Amino protecting groups useful in the present invention include hydrocarbonoxycarbonyl groups, such as alkoxycarbonyl groups (e.g., tert-butyloxycarbonyl (Boc)), alkenyloxycarbonyl groups (e.g., allyloxycarbonyl (Alloc)), aralkyloxycarbonyl groups (e.g., benzyloxycarbonyl (Cbz), 9-fluorenylmethoxycarbonyl (Fmoc)), silyloxycarbonyl groups (e.g., trimethoxysilylethoxycarbonyl (Teoc)); acyl groups, including carbonyl groups (e.g., phthalimido (Pht), 5,5-dimethyl-cyclohexane-1,3-dione-2-ethylidene (Dde)), sulfonyl groups (e.g., p-toluenesulfonyl (Tos)), halogenated alkylacyl groups (e.g., trifluoroacetyl (Tfa)); and alkyl groups, including aralkyl groups, such as trityl (Trt), 2,4-dimethoxybenzyl (Dmb), p-methoxybenzyl (Pmb), and benzyl (Bn).

Hydroxyl protecting groups useful in the present invention include tert-butyl (tBu).

Carboxyl protecting groups useful in the present invention include tert-butyloxy (OtBu).

Amide protecting groups useful in the present invention include trityl (Trt).

In some embodiments, lysine Lys uses Boc, Mtt, Alloc or Dde as the protecting group for its side chain ε-amino group, tryptophan Trp uses Boc as a side chain amino protecting group, serine Ser uses tBu as the protecting group for the side chain hydroxyl , tyrosine Tyr uses tBu as the protecting group for the side chain hydroxyl , threonine Thr uses tBu as the protecting group for the side chain hydroxyl, aspartic acid Asp uses tert-butoxy OtBu as the protecting group for the side chain carboxyl, glutamic acid Glu uses tert-butoxy OtBu as the protecting group for the side chain carboxyl, and/or glutamine Gln uses Trt as the protecting group for the side chain amide. Glu (γ-OtBu) means that the carboxyl group at γ-position of the glutamic acid is coupled to a tert-butoxy group, which is used for synthesizing the peptide chain of the polypeptide compound of Formula AI; Glu (α-OtBu) means that the carboxyl group at α-position of the glutamic acid is coupled to a tert-butoxy group, which is used for modifying the side chain ε-amino group of lysine.

In some embodiments, as for the lysines at position 16, position 24, position 28 and position 40, the lysine to be chemically modified at the side chain ε-amino group use sMtt, Alloc or Dde as the protecting group for the side chain ε-amino, and the lysines subjected to no chemical modification at the side chain ε-amino group use Boc as the protecting group for the side chain ε-aminos.

In some embodiments, as for the lysines at position 16, position 24, position 28 and position 40, the lysine to be chemically modified at the side chain ε-amino group uses Mtt as the protecting group for the side chain ε-amino, and the lysines subjected to no chemical modification at the side chain ε-amino groups use Boc as the protecting group for the side chain ε-aminos.

In some embodiments, as for the lysine at position 24, Mtt is used as the protecting group for the side chain ε-amino, and as for the lysines at position 16, position 28 and position 40, Boc is used as the protecting group for the side chain ε-amino groups.

**Table 1 Protecting groups useful in the present invention**

| Type | Abbreviation | Name | Formula |
|---|---|---|---|
| Alkoxycarbonyl | Boc | t-butyloxy carbonyl | 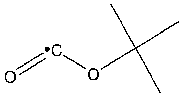 |
| Alkenyloxycarbonyl | Alloc | allyloxy carbonyl | 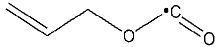 |
| Aralkyloxycarbonyl | Cbz | carbobenzyloxy | 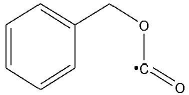 |
| | Fmoc | 9-fluorenyl mMethoxy carbonyl | 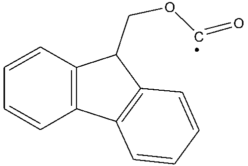 |
| Silaneoxycarbonyl | Teoc | 2-(trimethylsilyl) ethoxycarbonyl | 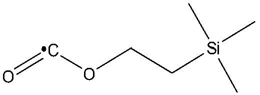 |
| Carbonyl | Pht | phthalimidyl | 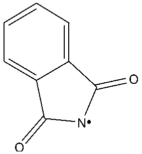 |
| | Dde | 2-Ethylidene-5,5-dimethylcyclohexane-1,3-dione | 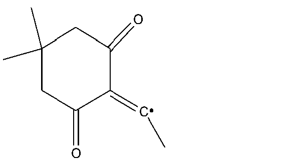 |
| Sulfonyl | Tos | p-toluene sulfonyl | 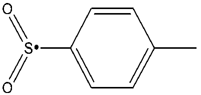 |
| Haloalkyl acyl | Tfa | Trifluoro acetyl | 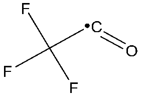 |
| Aralkyl | Trt | trityl | 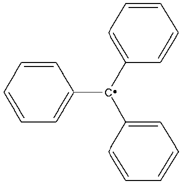 |
| | Mtt | 4-methyl trityl | 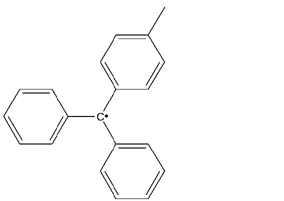 |
| | Dmb | 2,4-dimethoxy benzyl | 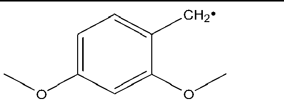 |
| | Pmb | para methoxy benzyl | 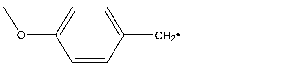 |
| | Bn | benzyl | 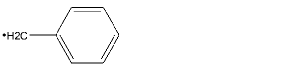 |
| alkyl | tBu | tert-butyl | 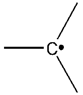 |
| Alkoxy | OtBu | tert-butoxy | 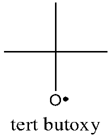 |

| | | | |
|---|---|---|---|
| Note: The dot "·" in the structures of the protecting groups represents the connection site at which the protecting groups attach to the groups of the amino acids that need protection. | | | |

The Boc solid-phase synthesis method in which the α-amino group is protected by a tert-butyloxycarbonyl (Boc), the Fmoc solid-phase synthesis method in which the α-amino group is protected by a 9-fluorenylmethoxycarbonyl (Fmoc), and the combination of the Boc solid-phase synthesis method and the Fmoc solid-phase synthesis method can be used in the present invention.

Taking Fmoc solid-phase synthesis method as an example, the peptide chain of the polypeptide compound of Formula AI can be prepared according to the reaction route as shown by the scheme below.

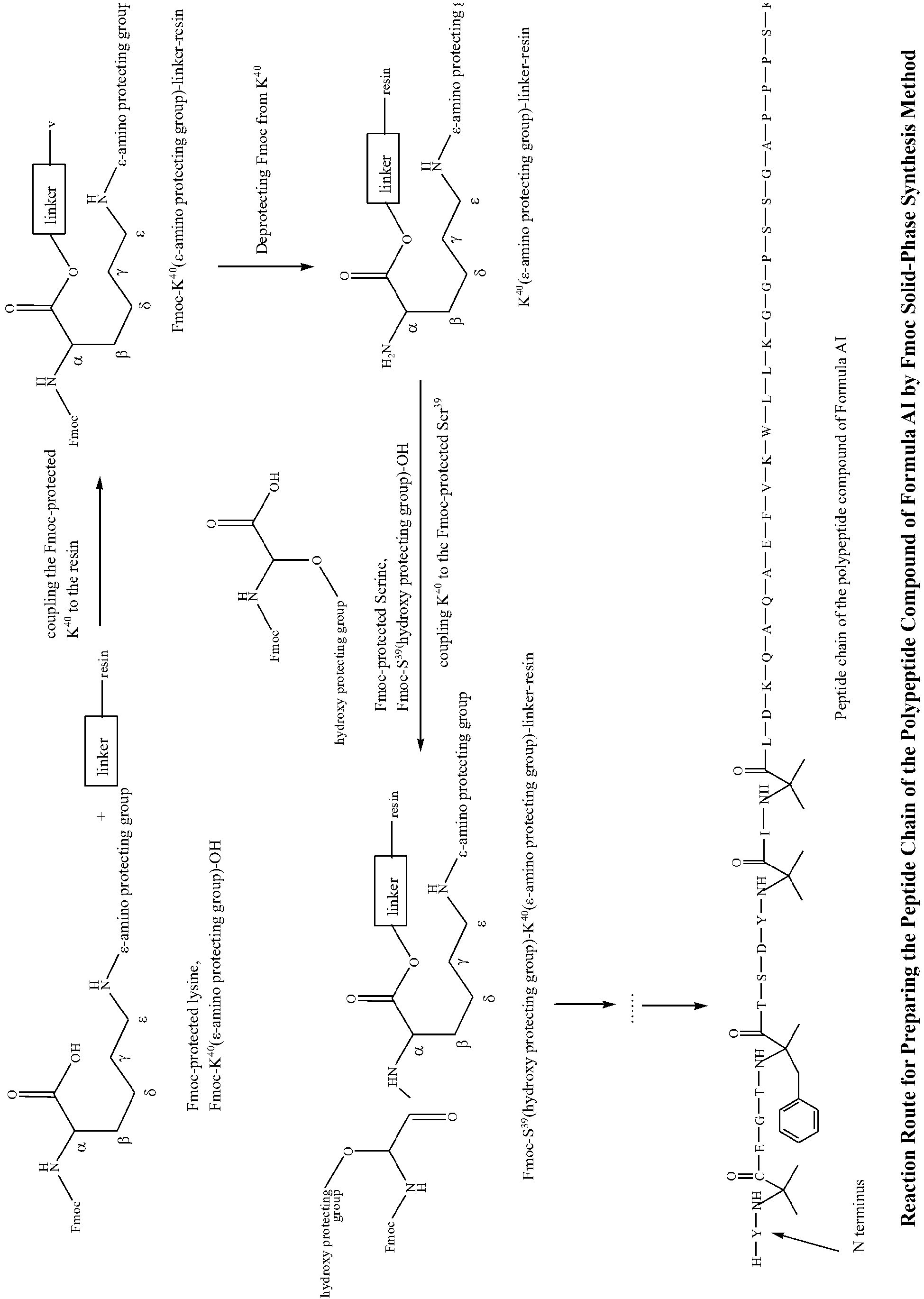

As shown above, according to the Fmoc solid-phase synthesis method, the C-terminal amino acid with α-amino protected by Fmoc is coupled to the resin support via a linker, and the Fmoc protecting group of the C-terminal amino acid coupled to the resin is removed. Starting from the rightmost C-terminus of the peptide chain of the polypeptide compound and heading toward the leftmost N-terminus, single amino acids and dipeptide fragments are sequentially coupled, wherein the carboxyl group of the amino acid to be coupled is condensed and coupled to the coupled amino group of the amino acid, wherein the amino acid to be coupled has a carboxyl group to be coupled and an amino group protected by Fmoc.

In some embodiments, when preparing the peptide chain of the polypeptide compound of Formula AI, the C-terminal amino acid (i.e., lysine K⁴⁰ at position 40) with α-amino protected by Fmoc is coupled to the resin support via a linker to obtain Fmoc-K⁴⁰ (ε-amino protecting group)-linker-resin. Then, the α-amino group of K⁴⁰ is deprotected to remove Fmoc to obtain K⁴⁰ (ε-amino protecting group)-linker-resin. The carboxyl group of the second amino acid from the C-terminus with α-amino protected by Fmoc (i.e., the amino acid to be coupled, serine S³⁹ at position 39) is coupled to the amino group of K⁴⁰ (i.e., the coupled amino acid) in K⁴⁰ (ε-amino protecting group)-linker-resin to obtain Fmoc-S³⁹ (hydroxyl protecting group)-K⁴⁰ (ε-amino protecting group)-linker-resin. In this way, starting from the rightmost C-terminus of the peptide chain of the polypeptide compound and heading toward the leftmost N-terminus, that is, along the direction from the 40^{th} amino acid to the 1^{st} amino acid (C-40→N-01), single amino acids and dipeptide fragments are sequentially condensed to obtain the peptide chain of the polypeptide compound of Formula AI

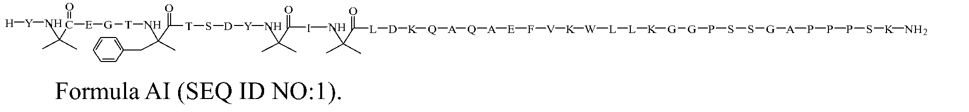

In the Boc solid-phase synthesis method, the reaction route is similar, except that the Fmoc protecting group used in the Fmoc solid-phase synthesis method is replaced by the Boc protecting group.

### [Dipeptide Fragment]

The polypeptide compound of Formula AI comprises 40 amino acid residues. In some embodiments, in the step of preparing the peptide chain of the polypeptide compound of Formula AI, the dipeptide fragments are selected from any of the following structures: Gly-Gly, Ile-Aib, Tyr-Aib, Thr-αMePhe and Glu-Gly. Each dipeptide fragment may appear one or more times.

In some embodiments, the dipeptide fragments are a combination of Gly-Gly, Ile-Aib and Tyr-Aib. Each dipeptide fragment may appear one or more times.

In some embodiments, the dipeptide fragments are a combination of Gly-Gly, Ile-Aib, Tyr-Aib, Thr-αMePhe and Glu-Gly. Each dipeptide fragment may appear one or more times.

In some embodiments, according to the solid-phase synthesis method via dipeptide fragments of the present invention, the polypeptide compound of Formula AI can be divided into 6 dipeptide fragments and 28 single amino acid fragments for 6 + 28 fragment coupling. In particular, the amino acids from position 1 to position 14 are divided into 9 fragments, and the amino acids from position 15 to position 40 are divided into 25 fragments. Each of the dipeptide fragments and single amino acid fragments are sequentially coupled from the C-terminus to the N-terminus. As compared with the 4+32 fragment coupling described hereinafter, the 6+28 fragment coupling can achieve a higher final yield and purity of crude product.

In some embodiments, said amino acids from position 1 to position 14 of the polypeptide compound of Formula AI can be divided into 9 fragments for coupling, in particular, the dipeptide fragments Tyr-Aib, Glu-Gly, Thr-αMePhe, Tyr-Aib, and Ile-Aib, and 4 single amino acid fragments other than the 5 dipeptide fragments; and the amino acids from position 15 to position 40 are divided into 25 fragments for coupling, in particular, a depeptide fragment Gly-Gly, and 24 single amino acid fragments other than the 1 dipeptide fragment, and each of the dipeptide fragments and single amino acid fragments are sequentially coupled from the C-terminus to the N-terminus.

In some embodiments, according to the solid-phase synthesis method via dipeptide fragments of the present invention, the polypeptide compound of Formula AI can be divided into 4 dipeptide fragments and 32 single amino acid fragments for 4 + 32 fragment coupling. In particular, the amino acids from position 1 to position 14 are divided into 11 fragments, and the amino acids from position 15 to position 40 are divided into 25 segments. Each of the dipeptide fragments and single amino acid fragments are sequentially coupled from the C-terminus to the N-terminus.

In some embodiments, said amino acids from position 1 to position 14 of the polypeptide compound of Formula AI can be divided into 11 fragments for coupling, in particular, the dipeptide fragments Tyr-Aib, Tyr-Aib, and Ile-Aib, and 8 single amino acid fragments other than the 3 dipeptide fragments; and the amino acids from position 15 to position 40 are divided into 25 fragments for coupling, in particular, a depeptide fragment Gly-Gly, and 24 single amino acids other than the 1 dipeptide fragment, and each of the dipeptide fragments and single amino acid fragments are sequentially coupled from the C-terminus to the N-terminus.

In some embodiments, in the step for preparing the peptide chain of the polypeptide compound of Formula AI, the peptide chain is divided into the following 6 fragments: Tyr¹-Aib², Glu³-Gly⁴, Thr⁵-αMePhe⁶, Tyr¹⁰-Aib¹¹, Ile¹²-Aib¹³, Gly²⁹-Gly³⁰, as well as the following single amino acids at 28 positions other than the 6 fragments: Thr⁷, Ser⁸, Asp⁹, Leu¹⁴, Asp¹⁵, Lys¹⁶, Gln¹⁷, Ala¹⁸, Gln¹⁹, Ala²⁰, Glu²¹, Phe²², Val²³, Lys²⁴, Trp²⁵, Leu²⁶, Leu²⁷, Lys²⁸, Pro³¹, Ser³², Ser³³, Gly³⁴, Ala³⁵, Pro³⁶, Pro³⁷, Pro³⁸, Ser³⁹ and Lys⁴⁰.

In some embodiments, in the step for preparing the peptide chain of the polypeptide compound of Formula AI, the peptide chain is divided into the following 4 fragments: Tyr¹-Aib², Tyr¹⁰-Aib¹¹, Ile¹²-Aib¹³, Gly²⁹-Gly³⁰, as well as the following single amino acids at the following 32 positions other than the 4 fragments: Glu³, Gly⁴, Thr⁵, αMePhe⁶, Thr⁷, Ser⁸, Asp⁹, Leu¹⁴, Asp¹⁵, Lys¹⁶, Gln¹⁷, Ala¹⁸, Gln¹⁹, Ala²⁰, Glu²¹, Phe²², Val²³, Lys²⁴, Trp²⁵, Leu²⁶, Leu²⁷, Lys²⁸, Pro³¹, Ser³², Ser³³, Gly³⁴, Ala³⁵, Pro³⁶, Pro³⁷, Pro³⁸, Ser³⁹ and Lys⁴⁰.

In some embodiments, the free amino group, in particular the free α-amino group of the dipeptide fragment, has an Fmoc protecting group, or a Boc protecting group, or both an Fmoc protecting group and a Boc protecting group. The free α-amino group of the dipeptide fragment refers to the nitrogen atom of the dipeptide fragment at α-position that does not form an amide bond, including primary amino groups -NH₂ and secondary amino groups -NH-.

In some embodiments, the dipeptide fragments having an Fmoc protecting group are selected from: Fmoc-Gly-Gly-OH, Fmoc-Ile-Aib-OH, Fmoc-Tyr(tBu)-Aib-OH, Fmoc-Thr(tBu)-αMePhe-OH and Fmoc-Glu(OtBu)-Gly-OH.

In some embodiments, the dipeptide fragments having a Boc protecting group are selected from: Boc-Gly-Gly-OH, Boc-Ile-Aib-OH, Boc-Tyr(tBu)-Aib-OH, Boc-Thr(tBu)-αMePhe-OH and Boc-Glu(OtBu)-Gly-OH.

In some embodiments, the dipeptide fragments having both an Fmoc protecting group and a Boc protecting group are selected from: Fmoc-Gly-Gly-OH, Fmoc-Ile-Aib-OH, Fmoc-Tyr(tBu)-Aib-OH, Fmoc-Thr(tBu)-αMePhe-OH, Fmoc-Glu(OtBu)-Gly-OH and Boc-Tyr(tBu)-Aib-OH.

In some embodiments, the dipeptide fragments having both an Fmoc protecting group and a Boc protecting group is a combination of Fmoc-Gly-Gly-OH, Fmoc-Ile-Aib-OH, Fmoc-Tyr(tBu)-Aib-OH, Fmoc-Thr(tBu)-αMePhe-OH, Fmoc-Glu(OtBu)-Gly-OH and Boc-Tyr(tBu)-Aib-OH.

The dipeptide fragments having both an Fmoc protecting group and a Boc protecting group means that in the same embodiment, a dipeptide fragment having an Fmoc protecting group and another dipeptide fragment having a Boc protecting group exist simultaneously, and does not mean that both the Fmoc protecting group and the Boc protecting group exists in the same dipeptide fragment.

In some embodiments, in the step of preparing the peptide chain of the polypeptide compound of Formula AI, the dipeptide fragments having both an Fmoc protecting group and a Boc protecting group is Boc-Tyr¹(tBu)-Aib²-OH, Fmoc-Glu³(OtBu)-Gly⁴-OH, Fmoc-Thr⁵(tBu)-αMePhe⁶-OH, Fmoc-Gly²⁹-Gly³⁰-OH, Fmoc-Tyr¹⁰(tBu)-Aib¹¹-OH and Fmoc-Ile¹²-Aib¹³-OH. The Boc protecting group is coupled to the amino group -NH₂ of the last coupled amino acid Tyr¹, which increases the stability of the peptide chain of the polypeptide compound of Formula AI as compared with the case where an Fmoc is coupled.

According to the present invention, when preparing the peptide chain of the polypeptide compound of Formula AI, starting from the rightmost C-terminus of the peptide chain and heading toward the leftmost N-terminus, that is, along the direction from the 40^{th} amino acid to the 1^{st} amino acid (C-40→N-01), the following dipeptide fragments and single amino acids are sequentially condensed (6 + 28 fragment coupling): Fmoc-Lys(Boc)-OH, Fmoc-Ser(tBu)-OH, Fmoc-Pro-OH, Fmoc-Pro-OH, Fmoc-Pro-OH, Fmoc-Ala-OH.H₂O, Fmoc-Gly-OH, Fmoc-Ser(tBu)-OH, Fmoc-Ser(tBu)-OH, Fmoc-Pro-OH, Fmoc-Gly-Gly-OH, Fmoc-Lys(Boc)-OH, Fmoc-Leu-OH, Fmoc-Leu-OH, Fmoc-Trp(Boc)-OH, Fmoc-Lys(Mtt)-OH, Fmoc-Val-OH, Fmoc-Phe-OH, Fmoc-Glu(γ-OtBu)-OH, Fmoc-Ala-OH.H₂O, Fmoc-Gln(Trt)-OH, Fmoc-Ala-OH.H₂O, Fmoc-Gln(Trt)-OH, Fmoc-Lys(Boc)-OH, Fmoc-Asp(OtBu)-OH, Fmoc-Leu-OH, Fmoc-Ile-Aib-OH, Fmoc-Tyr(tBu)-Aib-OH, Fmoc-Asp(OtBu)-OH, Fmoc-Ser(tBu)-OH, Fmoc-Thr(tBu)-OH, Fmoc-Thr(tBu)-αMePhe-OH, Fmoc-Glu(γ-OtBu)-Gly-OH, Boc-Tyr(tBu)-Aib-OH.

According to the present invention, when preparing the peptide chain of the polypeptide compound of Formula AI, starting from the rightmost C-terminus of the peptide chain and heading toward the leftmost N-terminus, that is, along the direction from the 40^{th} amino acid to the 1^{st} amino acid (C-40→N-01), the following dipeptide fragments and single amino acids are sequentially condensed (4 + 32 fragment coupling): Fmoc-Lys(Boc)-OH, Fmoc-Ser(tBu)-OH, Fmoc-Pro-OH, Fmoc-Pro-OH, Fmoc-Pro-OH, Fmoc-Ala-OH.H₂O, Fmoc-Gly-OH, Fmoc-Ser(tBu)-OH, Fmoc-Ser(tBu)-OH, Fmoc-Pro-OH, Fmoc-Gly-Gly-OH, Fmoc-Lys(Boc)-OH, Fmoc-Leu-OH, Fmoc-Leu-OH, Fmoc-Trp(Boc)-OH, Fmoc-Lys(Mtt)-OH, Fmoc-Val-OH, Fmoc-Phe-OH, Fmoc-Glu(γ-OtBu)-OH, Fmoc-Ala-OH.H₂O, Fmoc-Gln(Trt)-OH, Fmoc-Ala-OH.H₂O, Fmoc-Gln(Trt)-OH, Fmoc-Lys(Boc)-OH, Fmoc-Asp(OtBu)-OH, Fmoc-Leu-OH, Fmoc-Ile-Aib-OH, Fmoc-Tyr(tBu)-Aib-OH, Fmoc-Asp(OtBu)-OH, Fmoc-Ser(tBu)-OH, Fmoc-Thr(tBu)-OH, Fmoc-αMePhe-OH, Fmoc-Thr(tBu)-OH, Fmoc-Gly-OH, Fmoc-Glu(γ-OtBu)-OH, Boc-Tyr(tBu)-Aib-OH.

### [Modification of the Side Chain ε-Amino Group of Lysine ]

In some embodiments, in addition to preparing the peptide chain of the polypeptide compound of Formula AI by the solid-phase synthesis method via dipeptide fragments, the side chain ε-amino group of lysine is modified.

In some embodiments, after the peptide chain of the polypeptide compound of Formula AI is prepared by the solid-phase synthesis method via dipeptide fragments, the side chain ε-amino group of lysine is further modified.

In some embodiments, the modification of the side chain ε-amino group of lysine comprises: at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

In some embodiments, the modification of the side chain ε-amino group of lysine comprises:
as for the lysine using Mtt, Alloc or Dde as the protecting group for the side chain ε-amino group, the lysine is deprotected, and 2-(2-amino-ethoxy)-ethoxy]-acetyl in the number of a, γ-Glu in the number of b, and -CO-(CH₂)c-COOH in the number of 1 are sequentially coupled to the deprotected lysine ε-amino. Preferably, Mtt is used as the protecting group for the side chain ε-amino group of the lysine, since as compared with the case where Alloc or Dde is used as the protecting group for the side chain ε-amino group of the lysine, when Mtt is used as the protecting group of the side chain ε-amino group of the lysine, the final yield of the polypeptide compound of Formula AI can be improved, and the purity of the crude product are comparative to those obtained by using Alloc or Dde.

In some embodiments, 2-(2-amino-ethoxy)-ethoxy]-acetyl is coupled via Fmoc-AEEA-OH, γ-Glu is coupled via Fmoc-Glu(α-OtBu)-OH, and -OC-(CH₂)c-COOtBu is coupled via HOOC-(CH₂)c-COOtBu, and -OC-(CH₂)c-COOtBu is converted to -OC-(CH₂)c-COOH after post-treatment.

In some embodiments, the method for solid-phase synthesis of a polypeptide compound via dipeptide fragments comprises:
preparing the peptide chain of the polypeptide compound of Formula AI by the solid-phase synthesis method via dipeptide fragments,
as for the lysine using Mtt, Alloc or Dde as the protecting group for the side chain ε-amino group, the lysine is deprotected, and
at the side chain ε-amino group of the deprotected lysine, 2-(2-amino-ethoxy)-ethoxy]-acetyl is coupled via Fmoc-AEEA-OH, γ-Glu is coupled via Fmoc-Glu(α-OtBu)-OH, and -OC-(CH₂)c-COOtBu is coupled via HOOC-(CH₂)c-COOtBu, and
perforoming a post-treatment, and obtaining the polypeptide compound of Formula AI.

In some embodiments, the method for solid-phase synthesis of a polypeptide compound via dipeptide fragments comprises:
preparing the peptide chain of the polypeptide compound of Formula AI by the solid-phase synthesis method via dipeptide fragments, wherein the lysine at position 24 has an Mtt as the protecting group for the side chain ε-amino group, and the lysines at position 16, position 28 and position 40 have Bocs as the protecting groups for the side chain ε-amino groups;
deprotecting the side chain ε-amino group of the lysine at position 24; and
at the deprotected lysine at position 24, chemically modifying the side chain ε-amino group with ([2-(2-amino-ethoxy)-ethoxy]-acetyl)a-(γ-Glu)b-CO-(CH₂)c-COOH, thereby obtaining the polypeptide Compound P016.

In some embodiments, the method for solid-phase synthesis of a polypeptide compound via dipeptide fragments comprises:
preparing the peptide chain of the polypeptide compound of Formula AI by the solid-phase synthesis method via dipeptide fragments; wherein the lysine at position 24 has an Mtt as the protecting group for the side chain ε-amino group, and the lysines at position 16, position 28 and position 40 have Bocs as the protecting groups for the side chain ε-amino groups;
deprotecting the side chain ε-amino group of the lysine at position 24;
at the deprotected side chain ε-amino group of the lysine at position 24, chemically modifying the side chain ε-amino group of the lysine at positions 24 with ([2-(2-amino-ethoxy)-ethoxy]-acetyl)₃-(γ-Glu)₁-CO-(CH₂)₂₀-COOH by coupling a 2-(2-amino-ethoxy)-ethoxy]-acetyl) via Fmoc-AEEA-OH, coupling a γ-Glu via Fmoc-Glu(α-OtBu)-OH, and coupling a - OC-(CH₂)c-COO-tBu via HOOC-(CH₂)c-COOtBu;
   and
performing a post-treatment, thereby obtaining the polypeptide Compound P016.

### [Reaction Conditions for Solid-Phase Synthesis]

Prior to preparing the peptide chain, the C-terminal amino acid is firstly coupled to the resin as a support via its carbonyl group. The resin useful in the present invention is not particularly limited as long as it has the ability to bind to the reactive carbonyl group and is not sensitive to the reagents and reaction conditions for the segmented condensation and coupling as well as deprotection reactions. Examples of such resins include amino-type resins such as MBHA resins and Sieber resins; halogenated methyl resins such as chloromethyl resins, particularly chloromethyl-polystyrene-divinylbenzene polymers, and bromomethyl resins, etc.; hydroxymethyl resins; phenol resins; tert-butoxy-carbonylhydrazided resins; and crosslinked poly-N-allylacyl-pyridine resins etc.

In some embodiments, the resin is an amino-type resin, such as MBHA resin and Sieber resin.

In some embodiments, the resin has a low substitution value, preferably a substitution value of less than about 5 mmol/g, more preferably a substitution value of less than about 2 mmol/g, even more preferably a substitution value of less than about 1 mmol/g, still more preferably a substitution value of less than about 0.6 mmol/g, or more preferably a substitution value of about 0.3 to about 0.65 mmol/g.

In some embodiments, the resin is swelled prior to coupling to the C-terminal amino acid.

The swollen resin has a larger network space, allowing the reactant reagent molecules to enter the interior of the resin to react with functional groups and accommodate the continuously growing peptide chain. Useful swelling agent comprises the following organic solvents: dichloromethane (DCM), LDCM, N-methylpyrrolidone (NMP), 2-isopropylethylamine (DIEA), methyl tert-butyl ether (MTBE), trifluoroacetic acid (TFA), or any combination thereof, such as DIEA/DCM.

In some embodiments, useful coupling agent (i.e., condensing agent) comprises: N,N'-diisopropylcarbodiimide (DIC), 1-hydroxybenzotriazole (HOBt), 1-hydroxy-7-azobenzotriazole (HOAt), 2-(1H-benzotriazol-1-yl)-1,1,3,3-tetramethyluronium hexafluorophosphate (HBTU), 6-chloro-1-hydroxybenzotriazole (Cl-HOBt), or any combination thereof.

In some embodiments, the coupling agent is optionally used in combination with a suitable solvent (such as N,N- dimethylformamide (DMF)), for example, in the form of the following combinations: DIC/HOBt, HOBt/DMF, DIC/HOAt, HBTU/Cl-HOBt, DMF/DIC/HOBt, and DMF/DCM/DIC/HOBt.

In some embodiments, the condensation temperature is 5±5°C to 28±5°C, preferably 10±5°C to 25±5°C, for example 0-33°C, including both endpoints and any value and subrange within the range of 0-33°C, for example 0°C, 5°C, 10°C, 15°C, 20°C, 23°C, 30°C or 33°C.

In some embodiments, the condensing agent is used in an amount of about 2.0 to about 4.0 molar equivalents, relative to the amount of each amino acid or dipeptide to be coupled.

In some embodiments, the deprotecting agent comprises piperidine (PIP), hexafluoroisopropanol (HFIP), or a combination thereof.

In some embodiments, the deprotecting agent is optionally used in combination with a suitable solvent (e.g., DMF or DCM), for example, in the form of a combination of PIP/DMF or HFIP/DCM.

In some embodiments, after the modification of the side chain ε-amino group of the lysine, a post-treatment is further performed, including resin cleavage, product purification and lyophilization, so as to obtain a pure polypeptide compound.

In some embodiments, the cleavage agent comprises: trifluoroacetic acid (TFA), 1,2-ethanedithiol (EDT), triisopropylsilane (TIS), DODT, phenol (ArOH), MPR, ArSCH₃, or any combination thereof; preferably, a cleavaging system composed mainly of TFA, wherein the "cleavaging system composed mainly of TFA" means that TFA accounts for about 80 to about 95% by volume, EDT, TIS, DODT, ArOH, MPR and/or ArSCH₃ accounts for about 1 to about 10 % by volume, and the balance is H₂O, for example,
TFA:H₂O:EDT:TIS=about 94.0:2.0:2.0:2.0(V:V:V:V)
TFA:H₂O:DODT:TIS=about 94.0:2.0:2.0:2.0(V:V:V:V)
TFA:H₂O:EDT:TIS:ArOH =about 92.0:2.0:2.0:2.0:2.0(V:V:V:V:V)
TFA:H₂O:TIS:EDT:ArOH:ArSCH₃=about 81.5:5:1:2.5:5:5(V:V:V:V:V:V)
TFA:TIS:H₂O:MPR=about 94:2.5:2.5:1(V:V:V:V)
TFA:H₂O:EDT:TIS:ArOH=about 86.5:5:2.5:1:5(V:V:V:V:V);
preferably, for example,
TFA:H₂O:EDT:TIS:ArOH =about 92.0:2.0:2.0:2.0:2.0(V:V:V:V:V)
TFA:H₂O:TIS:EDT:ArOH:ArSCH₃=about 81.5:5:1:2.5:5:5(V:V:V:V:V:V);
TFA:H₂O:TIS:ArOH:ArSCH₃=about 84:5:1: 5:5(V:V:V:V:V),
wherein V represents that the ratio is volumn ratio.

In some embodiments, the dried polypeptide compound-resin is cut with a TFA cleavage system, filtered, concentrated, precipitated, and filtered to obtain a cleavage intermediate of the polypeptide compound AI with the resin support removed. Then, HPLC is used for purification and separation, and the fractions are collected, sorted, salt exchanged, and concentrated to obtain the purified intermediate of the polypeptide compound of Formula AI, which is then freeze-dried to obtain the polypeptide compound of Formula AI.

Those skilled in the art would appreciate that reaction reagents such as solubilizers, condensing agents, deprotecting agents, cleavage agents, solvents, and the like may be applied (such as complexing, mixing, and diluting, etc.) according to the methods commonly known in the art.

### EXAMPLES

### Example 1: Segmented Coupling (6+28 Fragment Coupling) for Polypeptide Compound P016

### [Materials for Reaction: Amino Acids, Dipeptide Fragments and Resin]

**Table 2 Amino acid fragments and resins**

| No. | Material Name | Molecular weight | position of the amino acid in the peptide chain |
|---|---|---|---|
| R-00 | MBHA Resin(s=0.3 to 0.65mmol/g) | | |
| R-01 | Fmoc-Nle-OH | 353 | |
| R-02 | Fmoc-Rink Linker | 540 | |
| A-01 | Fmoc-Lys(Boc)-OH | 468 | K⁴⁰ |
| A-02 | Fmoc-Ser(tBu)-OH | 384 | S³⁹ |
| A-03 | Fmoc-Pro-OH | 337 | P³⁸ |
| A-04 | Fmoc-Pro-OH | 337 | P³⁷ |
| A-05 | Fmoc-Pro-OH | 337 | P³⁶ |
| A-06 | Fmoc-Ala-OH.H₂O | 329 | A³⁵ |
| A-07 | Fmoc-Gly-OH | 297 | G³⁴ |
| A-08 | Fmoc-Ser(tBu)-OH | 384 | S³³ |
| A-09 | Fmoc-Ser(tBu)-OH | 384 | S³² |
| A-10 | Fmoc-Pro-OH | 337 | P³¹ |
| A-12-11 | Fmoc-Gly-Gly-OH | 354 | G ³⁰ -G ²⁹ |
| A-13 | Fmoc-Lys(Boc)-OH | 468 | K ²⁸ |
| A-14 | Fmoc-Leu-OH | 353 | L ²⁷ |
| A-15 | Fmoc-Leu-OH | 353 | L ²⁶ |
| A-16 | Fmoc-Trp(Boc)-OH | 526 | W²⁵ |
| A-17 | Fmoc-Lys(Mtt)-OH | 625 | K²⁴ |
| A-18 | Fmoc-Val-OH | 339 | V²³ |
| A-19 | Fmoc-Phe-OH | 387 | F²² |
| A-20 | Fmoc-Glu(γ-OtBu)-OH | 426 | E²¹ |
| A-21 | Fmoc-Ala-OH.H₂O | 329 | A²⁰ |
| A-22 | Fmoc-Gln(Trt)-OH | 611 | Q¹⁹ |
| A-23 | Fmoc-Ala-OH.H₂O | 329 | A¹⁸ |
| A-24 | Fmoc-Gln(Trt)-OH | 611 | Q¹⁷ |
| A-25 | Fmoc-Lys(Boc)-OH | 468 | K¹⁶ |
| A-26 | Fmoc-Asp(OtBu)-OH | 412 | D¹⁵ |
| A-27 | Fmoc-Leu-OH | 353 | L¹⁴ |
| A-29-28 | Fmoc-Ile-Aib-OH | 439 | Aib¹³-I¹² |
| A-31-30 | Fmoc-Tyr(tBu)-Aib-OH | 545 | Aib¹¹-Y¹⁰ |
| A-32 | Fmoc-Asp(OtBu)-OH | 412 | D⁹ |
| A-33 | Fmoc-Ser(tBu)-OH | 384 | S⁸ |
| A-34 | Fmoc-Thr(tBu)-OH | 398 | T⁷ |
| A-36-35 | Fmoc-Thr(tBu)-αMePhe-OH | 559 | αMePhe⁶-T⁵ |
| A-38-37 | Fmoc-Glu(γ-OtBu)-Gly-OH | 483 | G⁴-E³ |
| A-40-39 | Boc-Tyr(tBu)-Aib-OH | 423 | Aib²-Y¹ |
| Side 1 | Fmoc-AEEA-OH | 386 | |
| Side 2 | Fmoc-AEEA-OH | 386 | |
| Side 3 | Fmoc-AEEA-OH | 386 | |
| Side 4 | Fmoc-Glu(α-OtBu)-OH | 426 | |
| Side 5 | Mono-tert-butyl docosanediate (HOOC-(CH₂)₁₈-COOtBu) | 427 | |

| | | | |
|---|---|---|---|
| "R" represents resin, "A" represents amino acid, and "Side" represents side chain. | | | |

### [Synthesis of Fmoc-Rink Linker-Nle-MBHA Resin]

### 1. Swelling and Condensation of Resin

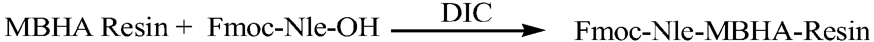

102.0 g of MBHA Resin as the starting resin was added to 0.7 L DCM. The resin was swelled under stirring for 30 minutes. The solution was drained, and the draining was repeated several times. A Ninhydrin colorimetric detection was performed, and the result showed that the swollen resin was colorless and transparent (the colorimetric detection was passed). Then, the solution of Fmoc-Nle-OH was added to a reaction bottle containing the swollen resin. During the reaction, a condensation agent DIC can be additionally added so as to complete the condensation reaction. After the condensation was completed, the solution was drained, and DMF was pumped into the bottle. The washing and draining were repeated several times to obtain the Fmoc-Nle-MBHA Resin.

### 2. De-protection of Resin

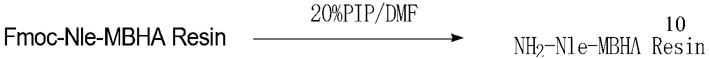

In a bottle containing piperidine, a PIP/DMF (V/V: 1:4) solution (de-protecting agent) was prepared. The solution was added to the reaction bottle, and reacted with the Fmoc-Nle-MBHA Resin under stirring for 30 minutes. The solution was drained, and the draining was repeated several times. A Ninhydrin colorimetric detection was performed, and the result showed that the resin was purple. The de-protected resin NH₂-Nle-MBHA-Resin was obtained.

### 3. Condensation of Resin

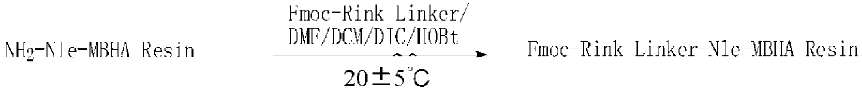

The solution of the Fmoc-Rink Linker was added to the reaction bottle containing the de-protected resin NH₂-Nle-MBHA-Resin. A Ninhydrin colorimetric detection was performed, and the results showed that the swollen resin was colorless and transparent (the colorimetric detection was passed), indicating that the condensation was complete. The solution was drained as much as possible, and DMF was pumped into the bottle. The washing and draining were repeated once or twice to obtain Fmoc-Rink Linker-Nle-MBHA Resin.

### [Preparation of the Peptide Chain of the Crude Peptide Compound P016 by Segmented Coupling]

### 1. De-protection of Resin

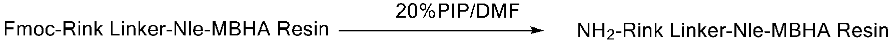

93.8 g of Fmoc-Lys(Boc)-OH and 31.0 g of condensing agent HOBt were added to a dissolution bottle containing DMF/DCM, the materials were cooled under stirring until completely dissolved. The solution was cooled in a cold bath at a temperature of ≤-5°C for more than 30 minutes, then a DIC/DCM solution was added slowly. After stirring, a Fmoc-Lys(Boc)-OH solution was obtained for further use.

In a bottle containing piperidine, a PIP/DMF (V/V: 1:4) solution was prepared. The solution was added to the drained Fmoc-Rink Linker-Nle-MBHA Resin. The mixture was stirred for 30 minutes, and the solution was drained. The washing and draining were repeated several times. A Ninhydrin colorimetric detection was performed, and the result showed that the resin was purple. The de-protected resin NH₂-Rink Linker-Nle-MBHA Resin was obtained.

### 2. Coupling of the C-Terminal Amino Acid (the 40^{th} Amino Acid FC⁴⁰ Starting from the Leftmost N-Terminal of the Peptide Chain)

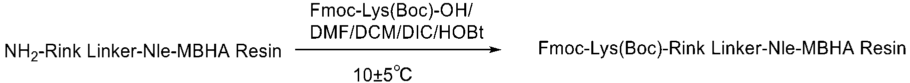

The solution of Fmoc-Lys(Boc)-OH was added to the reaction bottle containing the de-protected resin NH₂-Rink Linker-Nle-MBHA Resin. Acetic anhydride was diluted with DCM solution, and then DIEA was added and stirred homogeneously for condensation. The condensation was performed at a temperature of 10±5°C for 30 to 60 minutes. A Ninhydrin colorimetric detection was performed. If the result of the Ninhydrin detection showed that the resin was colorless or pale yellow, it indicated that there is no free amino group and the condensation is complete. Then, the solution was drained as much as possible, and DMF was pumped into the bottle and stirred for 2 minutes. The solution was drained. The washing and draining were repeated several times to obtain Fmoc-Lys(Boc)-Rink Linker-Nle-MBHA Resin.

Next, according to the sequence for coupling peptides as shown in Table 3 below, the above-mentioned steps were repeated, so as to couple the remaining 39 amino acids sequentially to the Fmoc-Lys(Boc)-Rink Linker-Nle-MBHA Resin until the coupling was completed, so as to obtain Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰ -Glu²¹(OtBu)-Phe²²- Val²³-Lys²⁴(Mtt)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻Ser³⁹(tBu)-Lys⁴⁰ (Boc)-Rink Linker-Nle-MBHA Resin (SEQ ID NO: 1).

**Table 3 Sequence for Coupling Peptide**

| Sequence for Coupling Peptide n= | Amino Acids or Dipeptide Fragments Having a Protecting Group | Position of the Amino Acid in the Peptide Chain |
|---|---|---|
| 1 | Fmoc-Ser(tBu)-OH | S³⁹ |
| 2 | Fmoc-Pro-OH | P³⁸ |
| 3 | Fmoc-Pro-OH | P³⁷ |
| 4 | Fmoc-Pro-OH | P³⁶ |
| 5 | Fmoc-Ala-OH | A³⁵ |
| 6 | Fmoc-Gly-OH | G³⁴ |
| 7 | Fmoc-Ser(tBu)-OH | S³³ |
| 8 | Fmoc-Ser(tBu)-OH | S³² |
| 9 | Fmoc-Pro-OH | P³¹ |
| 10 (dipeptide fragment) | Fmoc-Gly-Gly-OH | G³⁰-G²⁹ |
| 11 | Fmoc-Lys(Boc)-OH | K²⁸ |
| 12 | Fmoc-Leu-OH | L²⁷ |
| 13 | Fmoc-Leu-OH | L²⁶ |
| 14 | Fmoc-Trp(Boc)-OH | W²⁵ |
| 15 | Fmoc-Lys(Mtt)-OH | K²⁴ |
| 16 | Fmoc-Val-OH | V²³ |
| 17 | Fmoc-Phe-OH | F²² |
| 18 | Fmoc-Glu(γ-OtBu)-OH | E²¹ |
| 19 | Fmoc-Ala-OH | A²⁰ |
| 20 | Fmoc-Gln(Trt)-OH | Q¹⁹ |
| 21 | Fmoc-Ala-OH | A¹⁸ |
| 22 | Fmoc-Gln(Trt)-OH | Q¹⁷ |
| 23 | Fmoc-Lys(Boc)-OH | K¹⁶ |
| 24 | Fmoc-Asp(OtBu)-OH | D¹⁵ |
| 25 | Fmoc-Leu-OH | L¹⁴ |
| 26 (dipeptide fragment) | Fmoc-Ile-Aib-OH | Aib¹³-I¹² |
| 27 (dipeptide fragment) | Fmoc-Tyr(tBu)-Aib-OH | Aib¹¹-Y¹⁰ |
| 28 | Fmoc-Asp(OtBu)-OH | D⁹ |
| 29 | Fmoc-Ser(tBu)-OH | S⁸ |
| 30 | Fmoc-Thr(tBu)-OH | T⁷ |
| 31 (dipeptide fragment) | Fmoc-Thr(tBu)-αMePhe-OH | αMePhe⁶-T⁵ |
| 32 (dipeptide fragment) | Fmoc-Glu(γ-OtBu)-Gly-OH | G⁴-E³ |
| 33 (dipeptide fragment) | Boc-Tyr(tBu)-Aib-OH | Aib²-Y¹ |

### [Modification of Side Chain ]

### 1. De-protection of the side chain ε-amino group of the lysine Lys²⁴ at position 24

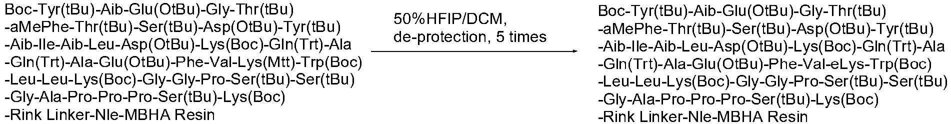

57.9 g of Fmoc-AEEA-OH and 23.3 g of HOBt were added to a dissolution bottle containing DMF. The solution was cooled under stirring until completely dissolved, and further cooled in a cold bath at a temperature of ≤ -5°C for more than 30 minutes, then a DIC/DMF solution was slowly added to the Fmoc-AEEA-OH/HOBt/DMF solution. After stirring, the Fmoc-AEEA- OH/HOBt/DIC/DMF solution was obtained for further use.

The formulated HFIP/DCM (V/V ratio 1:1) solution (de-protecting agent) was added to a reaction bottle containing Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(Mtt)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻ Ser³⁹(tBu)-Lys⁴⁰(Boc)-Rink Linker-Nle-MBHA Resin. The reactants were stirred for 30 minutes. The solution was drained. The washing and draining were repeated several times. A Ninhydrin colorimetric detection was performed, and the result showed that the resin was purple, indicating that the protecting group Mtt was removed at the side chain ε-amino group of the lysine Lys²⁴ at position 24 to obtain a peptide with the side chain ε-amino group de-protected.

### 2. Coupling Three AEEAs to the Side Chain ε-Amino Group of the Lysine Lys²⁴ at Position 24

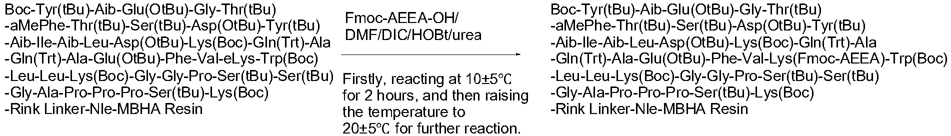

The solution of the Fmoc-AEEA-OH/HOBt/DIC/DMF was added to a reaction bottle containing the de-protected peptide chain. The condensation was performed at a temperature of 10±5°C for 2 hours. A Ninhydrin colorimetric detection was performed, and the result showed that the resin was colorless or pale yellow, indicating that the condensation was complete. The washing and draining were repeated several times to obtain a polypeptide compound with one AEEA coupled to the side chain ε-amino group of the lysine Lys²⁴at position 24: Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(Fmoc-AEEA)-Trp²⁵(Boc)-Leu²⁶-Leu²⁷-Lys²⁸(Boc)-Gly²⁹-Gly³⁰-Pro³¹-Se³²(tBu)-Ser³³(tBu)-Gly³⁴-Ala³⁵-Pro³⁶-Pro³⁷-Pro³⁸⁻Ser³⁹(tBu)-Lys⁴⁰(Boc)-Rink Linker-Nle-MBHA Resin.

The above de-protection and coupling AEEA were repeated more than twice to obtain a polypeptide compound with three AEEAs coupled to the side chain ε-amino group of the lysine Lys²⁴ at position 24: Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(Fmoc-AEEA-AEEA-AEEA)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻Ser³⁹(tBu)-Lys⁴⁰(Boc)-Rink Linker-Nle-MBHA Resin.

### 3. De-Protection of the Modification Group (Fmoc-AEEA-AEEA-AEEA) on the Side Chain ε-Amino Group of the Lysine Lys²⁴ at Position 24

The de-protection reaction was shown by the following Formula:

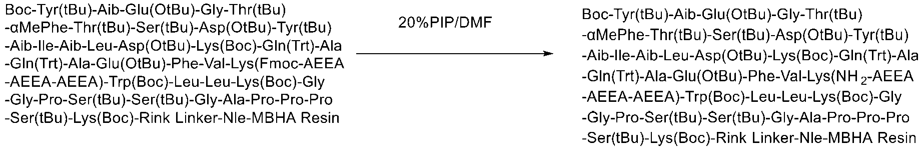

63.9 g of Fmoc-Glu(α-OtBu)-OH and 23.3 g of HOBt were added to a bottle containing DMF. The solution was cooled under stirring until completely dissolved, and further cooled in a cold bath at a temperature of ≤ -5°C for more than 30 minutes, then a DIC/DMF solution was slowly added to the Fmoc-Glu(α-OtBu)-OH/HOBt/DMF solution. After stirring, the Fmoc-Glu(α-OtBu)-OH/HOBt/DIC/DMF was obtained for further use.

The formulated PIP/DMF (V/V ratio 1:4) solution (de-protecting agent) was added to the polypeptide compound with three AEEAs coupled to the side chain ε-amino group of the lysine Lys²⁴ at position 24: Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(Fmoc-AEEA-AEEA-AEEA)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻Ser³⁹(tBu)-Lys⁴⁰(Boc)-Rink Linker-Nle-MBHA Resin. The reaction was conducted under stirring for 30 minutes. The solution was drained. The washing and draining were repeated several times. A Ninhydrin colorimetric detection was performed, and the result showed that the resin was purple, indicating that the protecting group Fmoc at (AEEA)₃ coupled to the side chain ε-amino group of the lysine Lys²⁴ at position 24 was removed to obtain a peptide with the (AEEA)₃ coupled to the side chain ε-amino group de-protected.

### 4. Coupling γ-Glu to (AEEA)₃ of the side chain ε-amino group of the lysine Lys²⁴ at position 24

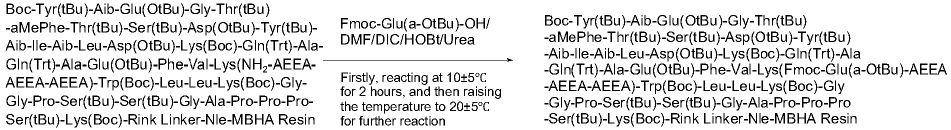

The solution of the Fmoc-Glu(α-OtBu)-OH/HOBt/DIC/DMF was added to a reaction bottle containing the peptide chain with the (AEEA)₃ coupled to the side chain ε-amino group de-protected. The reaction was performed at a temperature of 10±5°C for 2 hours. If necessary, DIC was additionally added to dilute by a factor of two for further condensation until a Ninhydrin colorimetric detection was performed, and the result showed that the resin was colorless or pale yellow, indicating that the condensation was complete. The solution was drained. The washing and draining were repeated several times to obtain a polypeptide compound with a γ-Glu coupled to (AEEA)₃ of the side chain ε-amino group of the lysine Lys²⁴ at position 24: Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(Fmoc-Glu(α-OtBu)-AEEA-AEEA-AEEA)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻Ser³⁹(tBu)-Lys⁴⁰(Boc)-Rink Linker-Nle-MBHA Resin.

### 5. De-protection at (AEEA)₃-γ-Glu of the ε-amino Group of the Side Chain of Lysine Lys²⁴ at position 24

The de-protection reaction was shown by the following Formula:

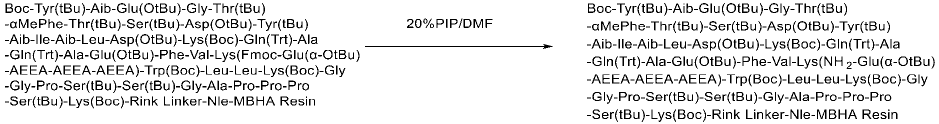

64.1 g of mono-tert-butyl docosanediate COOH-(CH₂)₂₀-COO-tBu and 23.3 g of HOBt were added to a bottle containing DMF. The DIC/DMF solution was then slowly added to the solution of COOH-(CH₂)₂₀-COO-tBu/HOBt/DMF. The reaction was performed at 20±5°C for more than 60 minutes to obtain COOH-(CH₂)₂₀-COO-tBu/HOBt/DIC/DMF for further use.

The formulated PIP/DMF (V/V ratio 1:4) solution (de-protecting agent) was added to the polypeptide compound with a γ-Glu coupled to (AEEA)₃ of the side chain ε-amino group of the lysine Lys²⁴ at position 24: Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(Fmoc-Glu(α-OtBu)-AEEA-AEEA-AEEA)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻Ser³⁹(tBu)-Lys⁴⁰(Boc)-Rink Linker-Nle-MBHA Resin. The reaction was conducted under stirring for 30 minutes. The solution was drained. The washing and draining were repeated several times. A Ninhydrin colorimetric detection was performed, and the results showed that the resin was purple, indicating that the protecting group Fmoc at (AEEA)₃-γ-Glu coupled to the side chain ε-amino group of the lysine at position 24 Lys²⁴ was removed to obtain a peptide chain with the de-protected (AEEA)₃-γ-Glu coupled to the side chain ε-amino group.

### 6. Coupling -CO-(CH₂)₂₀-COO-tBu to (AEEA)₃-γ-Glu of the Side Chain ε-amino Group of the Lysine Lys²⁴ at Position 24

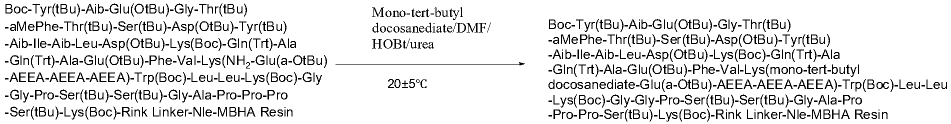

The solution of the COOH-(CH₂)₂₀-COO-tBu/HOBt/DIC/DMF was added to a reaction bottle containing the peptide chain with the de-protected (AEEA)₃-γ-Glu coupled to the side chain ε-amino group of the lysine 24 Lys²⁴at position. The synthesis reaction was performed at a temperature of 20±5°C for 2 hours. If necessary, DIC was additionally added to dilute by a factor of two for further condensation until a Ninhydrin colorimetric detection was performed, and the results showed that the resin was colorless or pale yellow, indicating that the condensation was complete. The solution was drained. The washing and draining were repeated several times to obtain a polypeptide compound with -CO-(CH₂)₂₀-COO-tBu coupled to (AEEA)₃-γ-Glu of the side chain ε-amino group of the lysine at position 24 Lys²⁴: Boc-Tyr¹(tBu)-Aib²-Glu³(OtBu)-Gly⁴-Thr⁵(tBu)-αMePhe⁶-Thr⁷(tBu)-Ser⁸(tBu)-Asp⁹(OtBu)-Tyr¹⁰(tBu)-Aib¹¹-Ile¹²-Aib¹³-Leu¹⁴-Asp¹⁵(OtBu)-Lys¹⁶(Boc)-Gln¹⁷(Trt)-Ala¹⁸-Gln¹⁹(Trt)-Ala²⁰-Glu²¹(OtBu)-Phe²²-Val²³-Lys²⁴(CO-(CH₂)₂₀-COO-tBu-Glu(α-OtBu)-AEEA-AEEA-AEEA)-Trp²⁵(Boc)-Leu²⁶-Leu²⁷-Lys²⁸(Boc)-Gly²⁹-Gly³⁰-Pro³¹-Ser³²(tBu)-Ser³³(tBu)-Gly³⁴-Ala³⁵-Pro³⁶-Pro³⁷-Pro³⁸⁻Ser³⁹(tBu)-Lys⁴⁰(Boc)-Rink Linker-Nle-MBHA Resin.

### [Post-treatment]

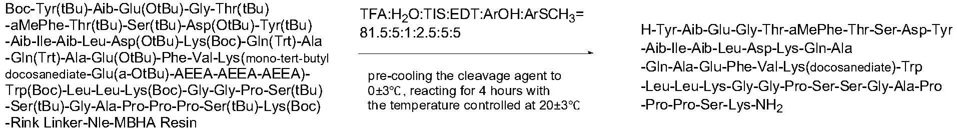

Under the protection of nitrogen, H₂O, TFA, ArOH, TIS, EDT, and ArSCH₃ were added sequentially and stirred homogenously. The formulated cleavage agent consisting of TFA:H₂O:EDT:TIS:ArOH:ArSCH₃ =81.5:5:2.5:1:5:5 (by volume) was slowly added to the resin. After cleavage, the resin was sufficiently stirred, and the solution was drained as much as possible to render the product fully dissolved in the cleavage reagent. When the reaction was completed, the reaction solution was filtered by suction, and then transferred to a bottle for concentration. The filtrate was concentrated to 20-45% of its initial volume under a reduced pressure at 32±2°C. The time for concentration was controlled to 30 to 60 minutes. The precooled methyl tert-butyl ether (MTBE) was added to the precipitation reaction bottle (vessel). The concentrated solution was added while stirring with a stirring bar. A white solid was precipitated. The liquid was filtrated by suction to obtain a cleavaged crude product in a form of white solid. After dissolved and de-carboxylated, and then filtered by a 0.45 µm organic membrane, the obtained cleavaged crude product was quantified.

### Preparation of Working Control Solution for Pure Peptide Amount and Purity:

The working control solution for the polypeptide compound of the present invention (for determining the pure peptide amount and purity) was prepared as follows:
precisely taking an appropriate amount of the working control of the polypeptide compound of the present invention, diluting it with purified water to prepare a solution containing about 0.50 mg/ml of the working control, and then shaking homogenously to serve as the working control solution.

### Method for Measuring the Samples:

Using full-load method, 50 µL of the working control solution of the polypeptide compound of the present invention, as well as the solutions for testing the pure peptide amount and purity were precisely taken using a 20 µL sampling loop. The solution was injected into a High-Performance Liquid Chromatography (HPLC) and measured according to the procedures recited in the 0512 General Chapters of the Fourth Part of the Chinese Pharmacopoeia (2020 Edition).

It was characterized by ESI-MS (electrospray ionization mass spectrometry) that the molecular weight of the synthesized polypeptide compound P016 was 5198.4.

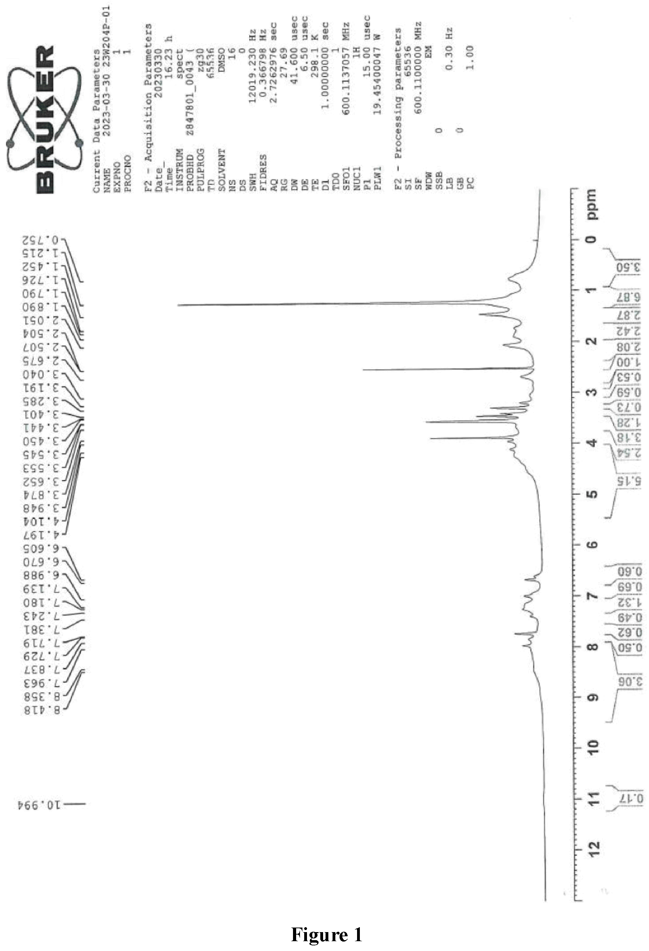

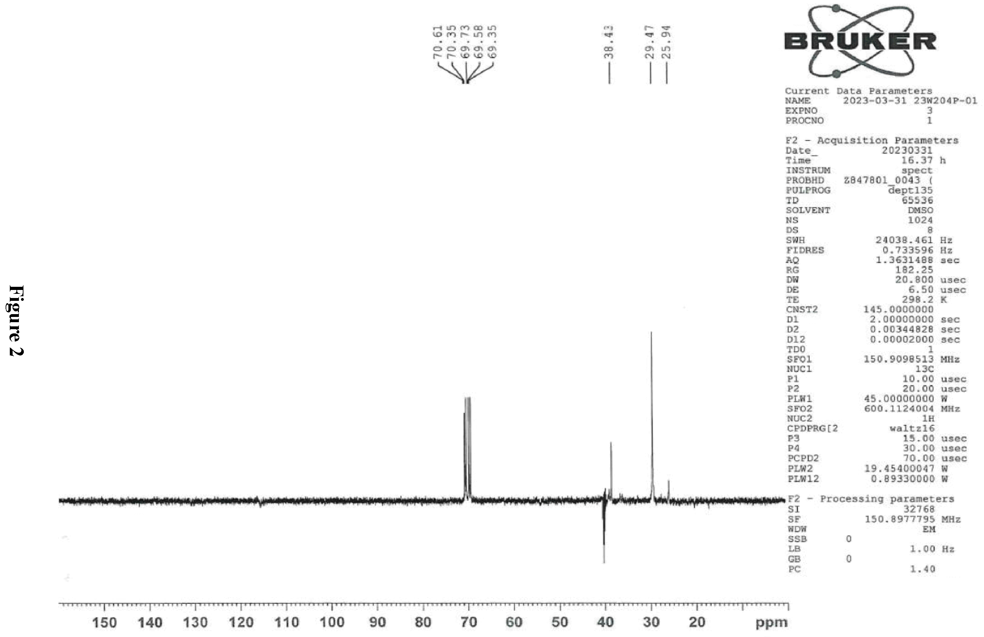

Figure 1 is the H-NMR spectrum of Compound P016. Figure 2 is the C-NMR spectrum of Compound P 016.

### [Example 2] Stepwise Synthesis Method

The polypeptide Compound P016 was prepared according to a method similar to Example 1, except that single amino acids were used instead of all the dipeptide fragments in Example 1.

### [Example 3] Dipeptide Fragment Synthesis Method (4+32 Fragment Coupling)

The polypeptide Compound P016 was prepared according to a method similar to Example 1, except that single amino acids were used instead of the dipeptide fragments Glu³-Gly⁴ and Thr⁵-αMePhe⁶ in Example 1.

### [Example 4] Large Fragment Synthesis Method (1+26 Fragment Coupling)

The polypeptide Compound P016 was prepared according to a method similar to Example 1, except that as for the amino acids starting from the 40^{th} amino acid to the 15^{th} amino acid (15 ← 40), the stepwise synthesis method was applied; as for the amino acids starting from the 14^{th} amino acid to the 1^{st} amino acid, a large fragment Boc-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴ was used instead of single amino acids and dipeptide fragments.

### [Example 5] Dipeptide Fragment Synthesis Method (6+28 Fragment Coupling)

The polypeptide Compound P016 was prepared according to a method similar to Example 1, except that Alloc was used instead of Mtt in Example 1 as the ε-amino protecting group of the lysine at positions 24.

### [Example 6] Dipeptide Fragment Synthesis Method (6+28 Fragment Coupling)

The polypeptide Compound P016 was prepared according to a method similar to Example 1, except that Dde was used instead of Mtt in Example 1 as the ε-amino protecting group of the lysine at positions 24.

### [Example 7] Dipeptide Fragment Synthesis Method (6+28 Fragment Coupling)

The polypeptide Compound P016 was prepared according to a method similar to Example 1, except that after the peptide chain was synthesized, firstly the moiety for modifying the side chain COOH-(CH₂)₂₀-COO-tBu-Glu(αOtBu)-AEEA-AEEA-AEEA was formed, and secondly the moiety for modifying the side chain COOH-(CH₂)₂₀-COO-tBu-Glu(αOtBu)-AEEA-AEEA-AEEA as a whole were directly coupled to the ε-amino group of lysine at position 24 of the peptide chain.

The methods for preparing the polypeptide compound P016 in Examples 1-6 and the test results of the products were recorded in Table 4 below.

**Table 4 Methods for Preparing the Polypeptide Compound P016 and the Test Results of the Products**

| No. | Example 1 | Example 2 | Example 3 | Example 4 | Example 5 | Example 6 | Example 7 |
|---|---|---|---|---|---|---|---|
| Stepwise/Fra gment Method | 15←28,31←40 | 1←40 | 15←28,31←40 | 15←40 | 15←28,31←40 | 15←28,31←40 | 15←28,31←40 |
| Large Fragment Synthesis Method | | | | 1-14 | | | |
| Position of Dipeptide Fragments | 1-2 | - | 1-2 | - | 1-2 | 1-2 | 1-2 |
| | 3-4 | | | | 3-4 | 3-4 | 3-4 |
| | 5-6 | | 10-11 | | 5-6 | 5-6 | 5-6 |
| | 10-11 | | 12-13 | | 10-11 | 10-11 | 10-11 |
| | 12-13 | | 29-30 | | 12-13 | 12-13 | 12-13 |
| | 29-30 | | | | 29-30 | 29-30 | 29-30 |
| Specific Embodiment | Dipeptide Fragment Synthesis Method (6+28 Fragment Coupling) (Boc-Tyr(tBu)-Aib-OH**, Fmoc-Glu(OtBu)-Gly-OH, Fmoc-Thr**(tBu)-**aMephe-OH**,Fmoc-Tyr(tBu)-Aib-OH ,Fmoc-Ile-Aib-OH)+[15←40]-Rink linker Nle MBHA Resin, wherein the amino acid at position 24 i**s: Fmoc-Lys(Mtt)-OH** | **Stepwise Synthesis Method**, wherein the amino acid at position 24 is: Fmoc-Lys(Mtt)-OH | Dipeptide Fragment Synthesis Method (4+32 Fragment Coupling)(Boc-Tyr(tBu)-Aib-OH,Fmoc-Tyr(tBu)-Aib -OH,Fmoc-Ile-Aib-OH)+[15←40]-Rink linker Nle MBHA Resin, wherein the amino acid at position 24 is: Fmoc-Lys(Mtt)-OH | Large Fragment Synthesis Method (1+26 Fragment Coupling) **[1-14]+[15**←**40]-**Rink linker-Nle-MBHA Resin, wherein the amino acid at position 24 is**:** Fmoc-Lys(Mtt)-OH | Dipeptide Fragment Synthesis Method (6+28 Fragment Coupling) (Boc-Tyr(tBu)-Aib-OH, Fmoc-Glu(OtBu)-Gly-OH, Fmoc-Thr(tBu)-aMephe-OH ,Fmoc-Tyr(tBu)-Aib-OH ,Fmoc-Ile-Aib-OH)+[15←40]-Rink linker Nle MBHA Resin, wherein the amino acid at position 24 is**: Fmoc-Lys(Alloc)-OH** | Dipeptide Fragment Synthesis Method (6+28 Fragment Coupling) (Boc-Tyr(tBu)-Aib-OH, Fmoc-Glu(OtBu)-Gly-OH, Fmoc-Thr(tBu)-aMephe-OH ,Fmoc-Tyr(tBu)-Aib-OH ,Fmoc-Ile-Aib-OH)+[15←40]-Rink linker Nle MBHA Resin, wherein the amino acid at po**s**ition 24 is**: Fmoc-Lys(Dde)-OH** | Dipeptide Fragment Synthesis Method (6+28 Fragment Coupling)(Boc-Tyr(tBu)-Aib-OH, Fmoc-Glu(OtBu)-Gly-OH, Fmoc-Thr(tBu)-aMephe-OH ,Fmoc-Tyr(tBu)-Aib -OH,Fmoc-Ile-Aib-OH)+[15←40]-Rink linker Nle MBHA Resin, wherein the amino acid at position 24 is**:** Fmoc-Lys(Mtt)-OH protection group,**However, the moieties for modifying the side chain as a whole were directly coupled to the peptide chain.** |
| Synthesis Yield | 102.50% | 67.10% | 85.00% | 96.20% | 90.60% | 81.20% | 78.50% |
| Purity of Crude Product | 63.90% | 13.94% | 51.15% | 52.27% | 67.01% | 59.71% | 67.20% |
| Final Yield | 31.54% | 5.22% | 16.92% | 18.56% | 13.65% | 12.50% | 14.62% |

In Table 4 above, the synthesis yield is calculated by dividing the moles of the crude P016 by the moles of the resin. The moles of the crude P016 are calculated by dividing the mass of the crude P016 by the molecular weight of P016 (i.e., 5198.4). Since the crude P016 comprises impurities, the synthesis yield may exceed 100%.

Purity of the crude product refers to the purity of the cleavaged crude product.

The final yield is calculated by dividing the moles of the pure peptide P016 after HPLC purification by the moles of the resin. The moles of the pure peptide P016 are calculated by dividing the mass of the pure peptide P016 by the molecular weight of P016 (i.e., 5198.4).

In Table 4 above, 15←28 refers to coupling sequentially from the 28^{th} amino acid to the 15^{th} amino acid; 31←40 refers to coupling sequentially from the 40^{th} amino acid to the 31^{st} amino acid; 15←40 refers to coupling sequentially from the 40^{th} amino acid to the 15^{th} amino acid; 1←40 refers to coupling sequentially from the 40^{th} amino acid to the 1^{st} amino acid; 1-14 means that the peptide chain from the 1^{st} amino acid to the 14^{th} amino acid as a whole is considered as one fragment; 1-2, 3-4, 5-6, 10-11, 12-13, and 29-30 are all dipeptide fragments.

In the dipeptide fragment synthesis method (6 +28 fragment coupling) of Example 1, the peptide chain was firstly synthesized, and then the side chain ε-amino group of the lysine at positions 24 was modified. In the peptide chain synthesis, six dipeptide fragments, namely Tyr¹-Aib², Glu³-Gly⁴, Thr⁵-αMePhe⁶, Tyr¹⁰-Aib¹¹, Ile¹²-Aib¹³, and Gly²⁹-Gly³⁰ were used. By protecting the side chain ε-amino group of lysine at position 24 with an Mtt, both excellent finial yield and purity of the crude product can be achieved. It can be seen from Table 4 above that, Example 2 adopted stepwise synthesis method; Example 4 adopted large fragment condensation method; the fragment synthesis method in Example 3 (4+32 fragment coupling) merely used parts of the dipeptide fragments; Examples 5 and 6 used Alloc and Dde as the protecting group for the side chain ε-amino group of the lysine at position 24 respectively; in Example 7, the moiety for modifying the side chain was firstly synthesized, and then the moiety for modifying the side chain was directly coupled to the peptide chain as a whole. The final yields of Examples 2 to 7 were significantly lower than that of the dipeptide fragment synthesis method of Example 1. The purity of the crude polypeptide compound in Example 2 was very low. The purities of the crude polypeptide compound of Examples 3 and 4 were significantly lower than that of Example 1. The purities of the crude polypeptide compounds of Examples 5, 6 and 7 were comparative to that of Example 1.

### [Example 8] Scale-Up Experiments for the Dipeptide Fragment Synthesis Method

The polypeptide Compound P016 was prepared according to a method similar to Example 1, except that, based on the moles of the resin charged, Example 1 was scaled from 10 mmol to 50 mmol (batch in small-scale), 300 mmol (batch in pilot-scale) and 300 mmol (batch in GMP-scale). The test results were recorded in Table 5 below. The batch in pilot-scale and the batch in GMP-scale were performed under the same operation conditions, except that the GMP-scale batch was performed in a GMP plant with the operation parameters such as the temperature being controlled more precisely.

**Table 5 Results for the Scale-Up Experiments**

| Batch | Small-Scale Batch | Pilot-Scale Batch | GMP-Scale Batch |
|---|---|---|---|
| Reaction Scale | 50 mmol | 300 mmol | 300 mmol |
| Purity of Pure product | 98.79% | 99.19% | 99.57% |
| Final Yield | 26.84% | 28.0 % | 31.38% |

It can be seen from Table 5 above that the method of the present invention can be stably scaled up to a large scale of 50 mmol-300 mmol without sacrificing the final yield and purity of the pure product.

### [Biological Activity of the Polypeptide Compound P016 of the Present Invention]

### 1. In Vitro Binding Activity to Human GlP-1 and GIP Receptors

hGLP-1 and the compound of the present invention were dissolved in DMSO and stored at -80°C. 89 µL of membrane dissolved in binding buffer (50 mM Hepes, pH 7.4, 5 mM MgCl₂ , 5 mM EDTA, 0.005% TWEEN, 0.005% HSA) was transferred to a 96-well test plate (5 µg/well). The compound was diluted in DMSO, and then, 1 µL of the diluted compound or 100% DMSO was added to the test plate containing the membrane solution. 10 µL of [125I] GLP-1 was further added (the final concentration in the reaction was 0.15 nM). The test plate was left at room temperature for 90 minutes. The membrane complexes were harvested into a GF/B plate precoated with 0.5% PEI by using a Cell Harvester, and washed for 3 times with 500 µL of pre-cooled elution buffer (50 mM Hepes, pH 7.4, 500 mM NaCl) at 4°C. After drying at 37°C for 2 hours, 50 µL of scintillation fluid was added to each well. The plate was read with a MicroBeta2 scintillation counter after sealing and settling for at least 1 hour so as to determine the membrane bound radioligand level.

The absolute IC₅₀ concentration of the compound was derived by non-linear regression of the binding percent of [125I]GLP-1 *versus* the concentration of the compound added. The IC₅₀ concentration was converted to Ki (Ki was the inhibition constant) using the Cheng-Prusoff equation.

hGIP and the polypeptide compound of the present invention were dissolved in DMSO and stored at -80°C. 98 µL of membrane dissolved in binding buffer (50 mM HEPES pH 7.4, 5 mM MgCl₂, 1 mM CaCl₂, 0.1% BSA, 0.005% Tween-20) was transferred to a 96-well test plate (15 µg/well). The compound was diluted in DMSO, and then, 2 µL of the diluted compounds or 100% DMSO was added to the test plate containing the membrane solution. 100 µL of [125I]GIP was further added (the final concentration in the reaction was 0.0315 nM). The test plate was left at room temperature for 90 minutes. The membrane complexes were harvested into a GF/B plate precoated with 0.5% PEI by using a Cell Harvester, and washed for 3 times with 500 µL of pre-cooled elution buffer (50 mM Tris-HCl pH 7.4, 125 mM NaCl) at 4°C. After drying at 37°C for 2 hours, 50 µL of scintillation fluid was added to each well. The plate was read with a MicroBeta2 scintillation counter after sealing and settling for at least 1 hour so as to determine the membrane bound radioligand level.

The absolute IC₅₀ concentration of the compound was derived by non-linear regression of the binding percent of [125I]GIP *versus* the concentration of the compound added. The IC₅₀ concentration was converted to Ki (Ki was the inhibition constant) using the Cheng-Prusoff equation.

**Table 6. Receptor Binding Affinity of the Polypeptide**

| Compound P016 of the Present Invention, Ki Ratio | | |
|---|---|---|
| Compound | GLP-1R | GIPR |
| | Ki ratio | Ki ratio |
| GLP-1 | 1.00 | NA |
| GIP | NA | 1.00 |
| P001 | 19.52 | 8.82 |
| Tirzepatide | 0.28 | 0.06 |
| P007 | 0.47 | NA |
| P008 | 1.09 | 0.16 |
| P013 | 0.28 | 0.14 |
| P014 | 0.37 | 0.08 |
| P015 | 0.17 | NA |
| P016 | 0.63 | 0.11 |
| P017 | 0.56 | 0.04 |
| P018 | 1.45 | 0.05 |
| P019 | 0.93 | 0.09 |
| P020 | 1.36 | 0.05 |

| | | |
|---|---|---|
| Ki ratio: the ratio of the Ki value of the endogenous ligand to the Ki value of the test compound | | |

### 2. Agonistic Activity on hGLP-1R and hGIPR

The agonistic activities of the polypeptide compounds according to the present invention on human GLP-1R were determined by cAMP reporter gene assay.

HEK293/CRE/GLP-1R cells were inoculated in a 96-well plate at 50,000 cells/well (80 µL/well), and incubated overnight in a 37°C, 5% CO₂ incubator. 20 µL/well of the test medium (DMEM containing 0.1% casein) containing a compound was added to the 96-well plate, and continued to incubate in a 37°C, 5% CO₂ incubator for 6 hours and equilibrated to room temperature. The supernatant was removed. 50 µL/well Bright-Glo reagent was added, shaked and lysed at room temperature for 10 minutes. The luminescence was read using an Envision microplate reader, and the luciferase activity was measured.

The response value to 100 nM GLP-1 was set as 100% response value, and GraphPad was used to perform nonlinear regression according to the response percentage and the concentration of the compound added to obtain the EC₅₀ value for each compound.

The agonistic activities of the compounds according to the present invention on human GIPR were determined by a LANCE Ultra cAMP Kit.

The in vitro agonistic activities of the compounds according to the present invention on GIPR was determined in HEK293 cells stably expressing human GIPR (HEK293/GIPR cells). HEK293/GIPR cells were formulated in HBSS buffer (0.1% Casein, 500 µM IBMX, 5 mM HEPES), and inoculated into a 384-well cell culture plate at 1000 cells/well (5 µL/well). 5 µL of HBSS buffer containing compounds was added to the above-mentioned 384-well cell culture plate. After been sealed, the plate was placed into a 37°C, 5% CO₂ incubator for about 30 minutes. After the incubation was completed, 5 µL of cAMP-Eu working solution and 5 µL of cAMP-Ulight working solution were added sequentially, and the plate was shaken for uniform mixing. The incubation was performed for 1 hour at 25°C. The signal values at 665 nm and 615 nm were read on an Envision microplate reader. The ratio of the signal value at 665 nm to the signal value at 615 was calculated, and converted into a cAMP concentration by using the cAMP standard curve. The response value to 1 µM GIP was set as 100% response value, and GraphPad was used to perform nonlinear regression according to the response percentage and the concentration of the polypeptide compound of the present invention added to obtain the EC₅₀ value for each compound.

**Table 7. Agonist Activity on Human GLP-1 and GIP Receptors, EC₅₀ Values**

| Compound | GLP-1R | GIPR |
|---|---|---|
| | EC₅₀ value | EC₅₀ value |
| GLP-1 | 1.00 | NA |
| GIP | NA | 1.00 |
| P001 | 52.09 | 7.13 |
| Tirzepatide | 0.74 | 0.51 |
| P007 | 5.84 | 1.15 |
| P008 | 4.79 | 1.67 |
| P013 | 2.41 | 0.86 |
| P014 | 1.73 | 0.25 |
| P015 | 2.44 | 0.17 |
| P016 | 1.56 | 0.56 |
| P017 | 1.35 | 0.16 |
| P018 | 3.63 | 2.55 |
| P019 | 4.95 | 1.68 |
| P020 | 1.41 | 0.22 |

| | | |
|---|---|---|
| EC₅₀ value: the ratio of the EC₅₀ value of the endogenous ligand to the EC₅₀ value of the test compound | | |

### 3. Pharmacodynamics Effects in db/db Mice was intended to studying the effects of the polypeptide compounds according to the present invention on blood glucose in diabetic model mice (db/db mice).

In this study, db/db mice were subcutaneously single administered, and the changes in the blood glucose, the food intake, and the body weight of the mice were measured to illustrate the hypoglycemic effects of the compounds according to the present invention as well as the duration of the drug effect. In this study, 8-9 week old male db/db mice were used. The db/db mice were placed in an individual ventilated cage (IVC) facility with a controlled temperature (20-26°C) and humidity (40-70%) and a controlled 12h:12h light-dark cycle. The mice can receive food and water ad libitum. Blood was sampled at the tail tip, and the base blood glucose was measured by a Roche blood glucose meter. The mice were randomly divided into groups according to the initial blood glucose and initial body weight (n=6/group), and each group had similar body weight and blood glucose.

The compounds of the invention (10 nmol/kg) were dissolved in a solvent (PBS containing 0.1% Tween 20, pH 7.2-7.4). At the set time point (0 to 120 hours), the recorded hypoglycemic duration of the polypeptide compounds has a significant difference vesus the vehicle group.

**Table 8. Hypoglycemic Duration of the Polypeptide**

| Compounds of the Present Invention | | |
|---|---|---|
| Group | Compound | Hypoglycemic Duration (h) |
| | P001 | 32 |
| | Tirzepatide | 56 |
| Group 1 | P007 | 56 |
| | P008 | 72 |
| | P014 | 104 |
| | P019 | 56 |
| | Tirzepatide | 48 |
| | P013 | 72 |
| | P015 | 32 |
| Group 2 | P016 | 104 |
| | P017 | 72 |
| | P018 | 48 |
| | P020 | 104 |

The polypeptide compounds provided by the present invention and use thereof are described in detail above.

Specific examples were applied herein to illustrate the principle and embodiments of the present invention. The illustration of the above examples was merely used to help understand the method and the central idea of the present invention. It should be indicated that those skilled in the art can make several improvements and modifications to the present invention without departing from the principles of the present invention, and these improvements and modifications also fall within the protection scope of the claims of the present invention. Amino Acid Sequences:

| No. in sequence listing | Name | Sequence |
|---|---|---|
| (SEQ ID NO:1) | P001 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib (SEQ ID NO:1) |
| (SEQ ID NO:2) | P007 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₁-CO-(CH₂)₁₆-CO₂H (SEQ ID NO:2). |
| (SEQ ID NO:3) | P008 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; K at position 28 is chemically modified through conjugation to the ε-amino of the K side-chain with ([2-(2-amino-ethoxy)-ethoxy]-acetyl)₂-(γ-Glu)₁-CO-(CH₂)₁₆-CO₂H (SEQ ID NO:3). |
| (SEQ ID NO:4) | P013 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₁-CO-(CH₂)₁₈-CO₂H (SEQ ID NO:4). |
| (SEQ ID NO:5) | P014 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₁-CO-(CH₂)₂₀-CO₂H (SEQ ID NO:5). |
| (SEQ ID NO:6) | P015 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₂-(γ-Glu)₃-CO-(CH₂)₁₆-CO₂H (SEQ ID NO:6). |
| (SEQ ID NO:7) | P016 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 24 is chemically modified through conjugation to the ε-amino of the K side-chain with ([2-(2-amino-ethoxy)-ethoxy]-acetyl)₃-(γ-Glu)₁-CO-(CH₂)₂₀-CO₂H (SEQ ID NO:7). |
| (SEQ ID NO:8) | P017 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₂-(γ-Glu)₁-CO-(CH₂)₂₀-CO₂H (SEQ ID NO:8). |
| (SEQ ID NO:9) | P018 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₂-(γ-Glu)₃-CO-(CH₂)₁₆-CO₂H (SEQ ID NO:9). |
| (SEQ ID NO:10) | P019 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₃-(γ-Glu)₁-CO-(CH₂)₁₆-CO₂H (SEQ ID NO:10). |
| (SEQ ID NO:11) | P020 | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | K at position 28 is chemically modified through conjugation to the ε-amino of the K side-chain with ([2-(2-amino-ethoxy)-ethoxy]-acetyl)₃-(γ-Glu)₁-CO-(CH₂)₂₀-CO₂H (SEQ ID NO:11). |
| (SEQ ID NO:12) | Formula (AI) | Y-X1-E-G-T-X2-T-S-D-Y-X3-I-X4-L-D-K-Q-A-Q-A-E-F-V-K-W-L-L- K-G-G-P-S-S-G-A-P-P-P-S-K; wherein X₁ is Aib; X₂ is αMePhe; X₃ is Aib; X₄ is Aib; |
| | | optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24 (SEQ ID NO:12). |

## Claims

1. Use of dipeptide fragments in solid-phase synthesis of a polypeptide compound, **characterized in that** the dipeptide fragments are selected from the following structures: Gly-Gly, Ile-Aib, Tyr-Aib, Thr-αMePhe and Glu-Gly, and the polypeptide compound is a compound of Formula AI (SEQ ID NO:12):
H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI,
and has the following structure: 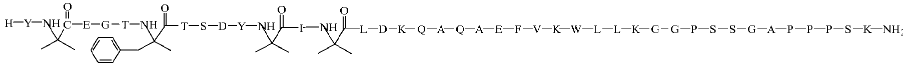
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

2. The use according to claim 1, **characterized in that** the polypeptide compound are selected from the following compounds:
Compound P001: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:1) 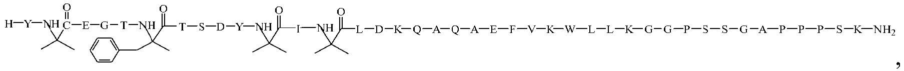
Compound P007: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²¹-Lys²⁴(AEEA-AEEA-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:2) 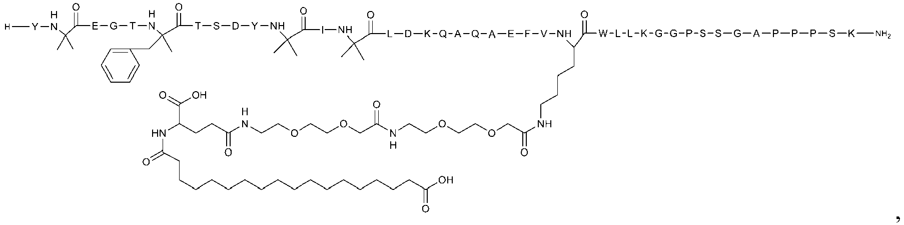
Compound P008: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:3) 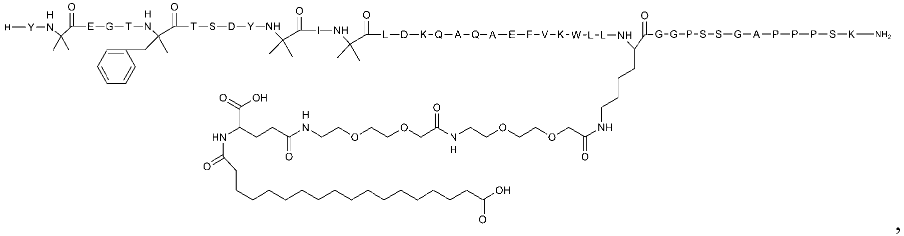
Compound P013: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₁₈-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:4) 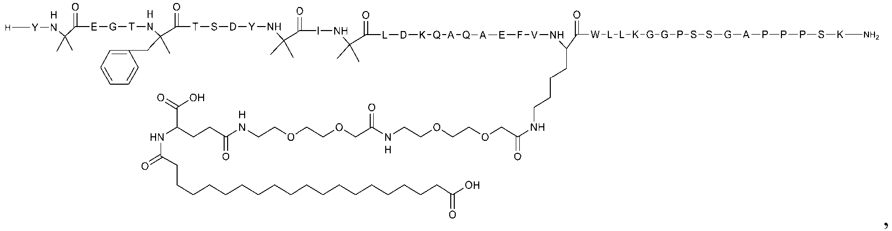
Compound P014: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:5) 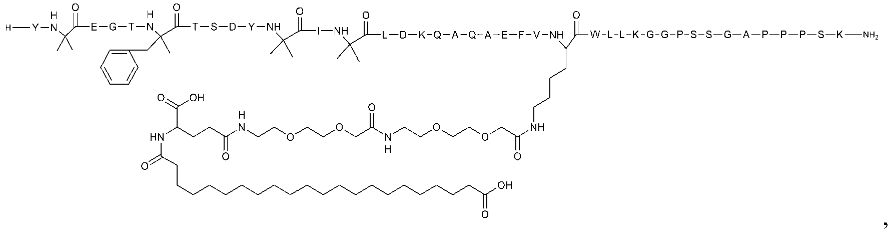
Compound P015: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-γGlu-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵ -Pro³⁶-Pro³⁷-Pro³⁸-Ser³⁹-Lys⁴⁰-NH₂ (SEQ ID NO:6) 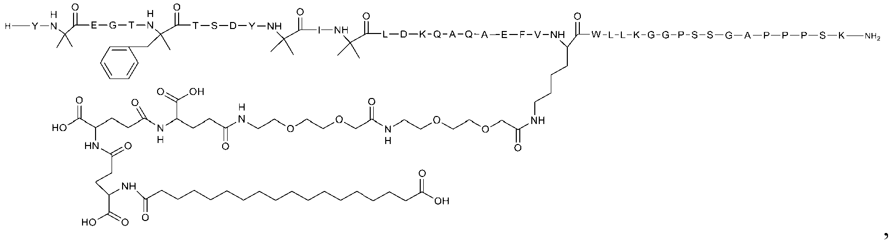
Compound P016: H-Tyr¹-Aib²-Glu³-Gly-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Prp³⁶-Pro³⁷-Pro³⁸-Ser³⁹-Lys⁴⁰-NH₂ (SEQ ID NO:7) 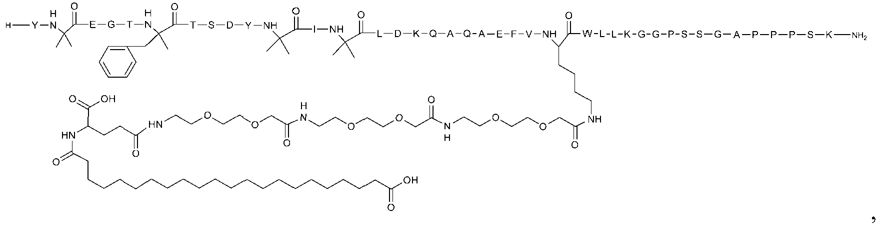
Compound P017: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:8) 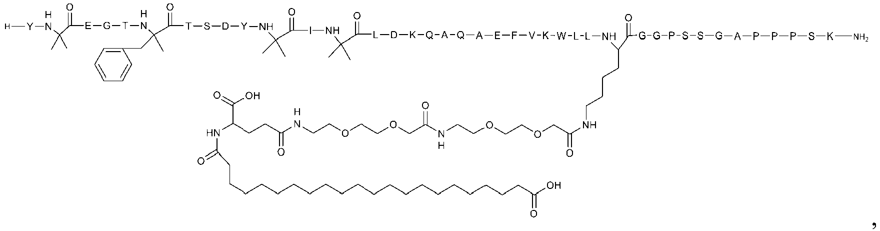
Compound P018: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-γGlu-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:9) 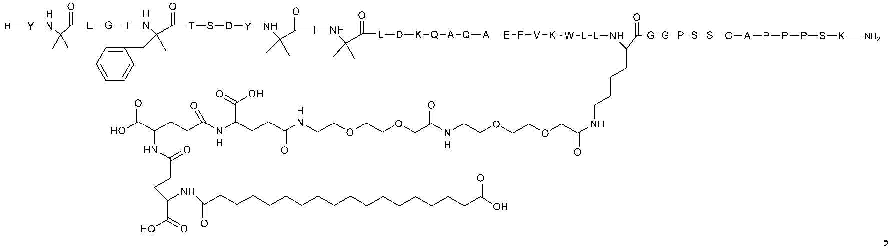
Compound P019: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO: 10) 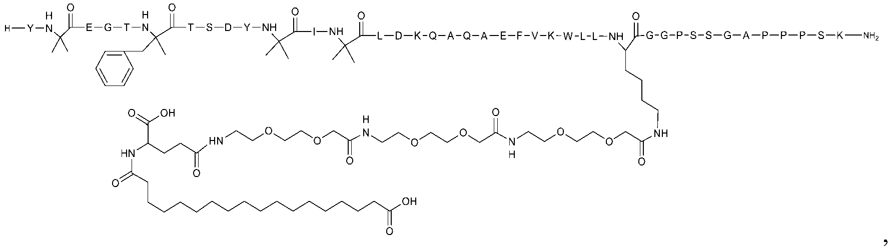 and
Compound P020: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA -γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO: 11) 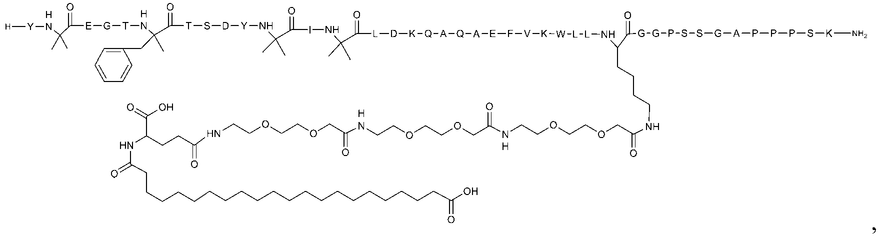
wherein AEEA represents [2-(2-amino-ethoxy)-ethoxy]-acetyl.

3. The use according to claim 1 or 2, **characterized in that** the polypeptide compound is:
Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:7)

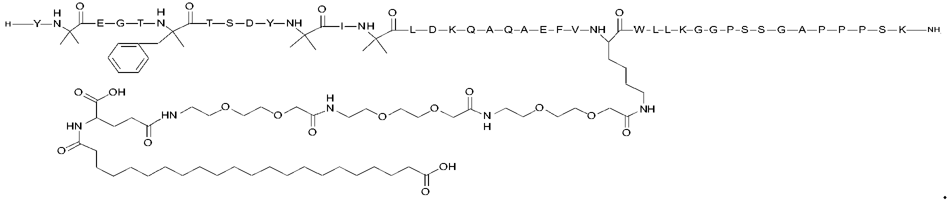

4. The use according to any one of claims 1 to 3, **characterized in that** the free α-amino group of the dipeptide fragment has an Fmoc protecting group, or a Boc protecting group, or both an Fmoc protecting group and a Boc protecting group.

5. The use according to claim 4, **characterized in that** the dipeptide fragment having an Fmoc protecting group is selected from: Fmoc-Gly-Gly-OH, Fmoc-Ile-Aib-OH, Fmoc-Tyr(tBu)-Aib-OH, Fmoc-Thr(tBu)-αMePhe-OH and Fmoc-Glu(OtBu)-Gly-OH.

6. The use according to claim 4, **characterized in that** the dipeptide fragment having a Boc protecting group is selected from: Boc-Gly-Gly-OH, Boc-Ile-Aib-OH, Boc-Tyr(tBu)-Aib-OH, Boc-Thr(tBu)-αMePhe-OH and Boc-Glu(OtBu)-Gly-OH, preferably Boc-Tyr(tBu)-Aib-OH.

7. The use according to claim 4, **characterized in that** the dipeptide fragment having both an Fmoc protecting group and a Boc protecting group is selected from: Fmoc-Gly-Gly-OH, Fmoc-Ile-Aib-OH, Fmoc-Tyr(tBu)-Aib-OH, Fmoc-Thr(tBu)-αMePhe-OH, Fmoc-Glu(OtBu)-Gly-OH and Boc-Tyr(tBu)-Aib-OH;
preferably, the dipeptide fragments having both an Fmoc protecting group and a Boc protecting group is a combination of Fmoc-Gly-Gly-OH, Fmoc-Ile-Aib-OH, Fmoc-Tyr(tBu)-Aib-OH, Fmoc-Thr(tBu)-αMePhe-OH, Fmoc-Glu(OtBu)-Gly-OH and Boc-Tyr(tBu)-Aib-OH.

8. The use according to any one of claims 1 to 7, **characterized in that** the polypeptide compound of Formula AI is divided into the following 6 fragments: Tyr¹-Aib², Glu³-Gly⁴, Thr⁵-αMePhe⁶, Tyr¹⁰-Aib¹¹, Ile¹²-Aib¹³, and Gly²⁹-Gly³⁰, as well as single amino acids at the remaining 28 sites for coupling.

9. A method for solid-phase synthesis of a polypeptide compound via dipeptide fragments, **characterized in that** the polypeptide compound is a compound of Formula AI,
H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵- Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰- Pro³¹-Ser³²-Ser³³-Gly³⁴-Ala³⁵-Pro³⁶-Pro³⁷-Pro³⁸⁻Ser³⁹-Lys⁴⁰-NH₂ Formula AI (SEQ ID NO:12),
and has the following structure: 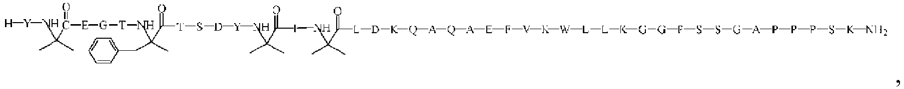
wherein optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24,
the dipeptide fragments are selected from the following structures: Gly-Gly, Ile-Aib, Tyr-Aib, Thr-αMePhe and Glu-Gly.

10. The method according to claim 9, wherein the polypeptide compound is a compound selected from:
Compound P001: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:1) 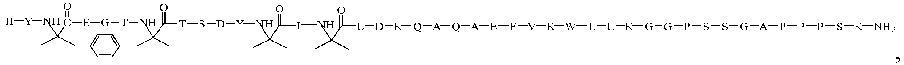
Compound P007: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:2) 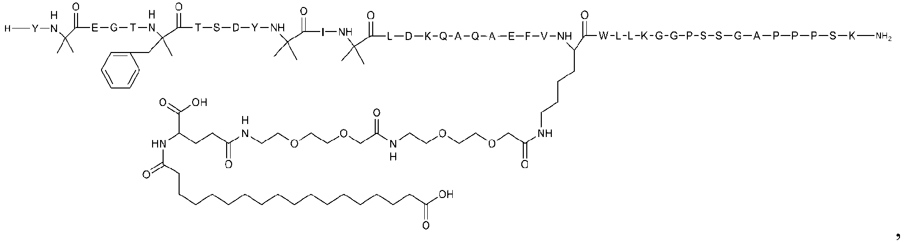
Compound P008: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:3) 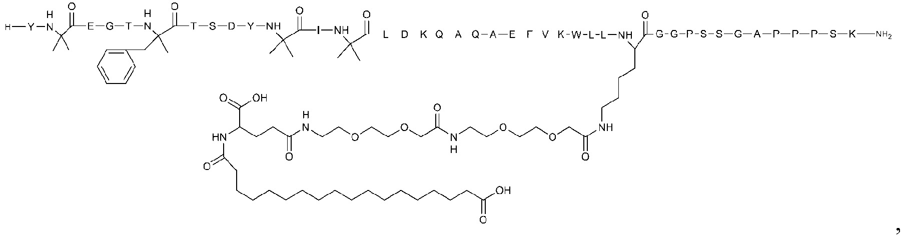
Compound P013: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₁₈-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:4) 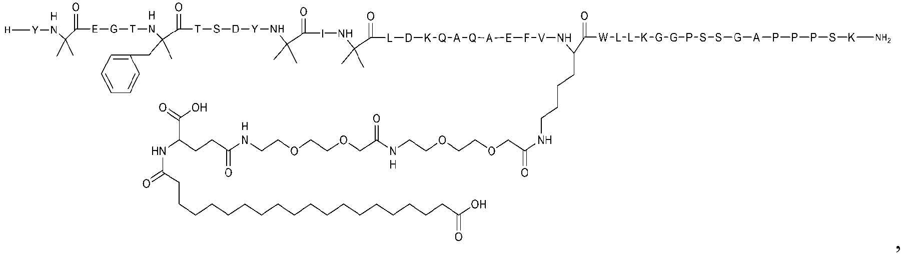
Compound P014: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:5) 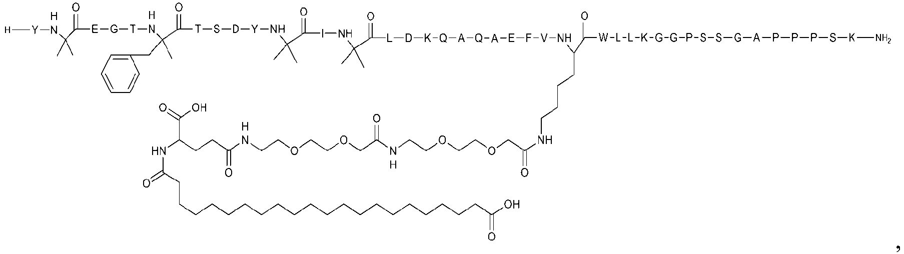
Compound P015: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-γGlu-γGlu-γGlu-CO-(CH₂)₁₆-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:6) 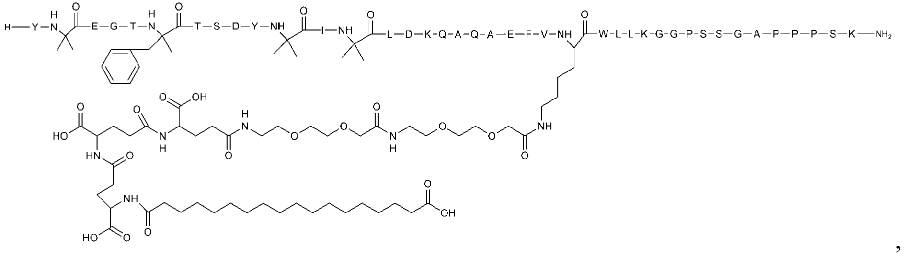
Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:7) 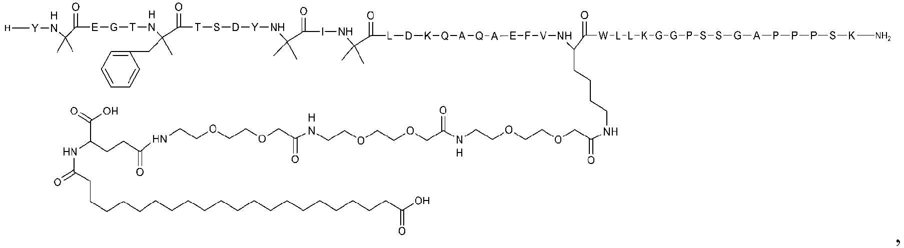
Compound P017: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:8) 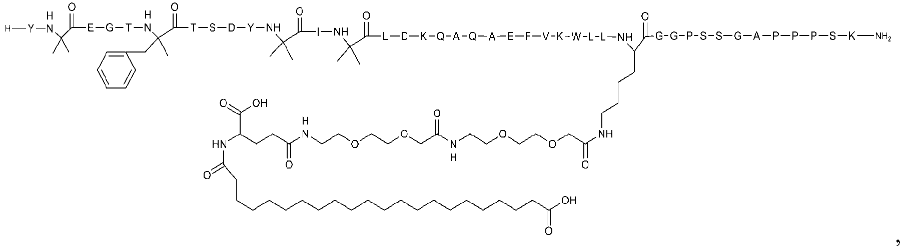
Compound P018: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-γGlu-γGlu-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO:9) 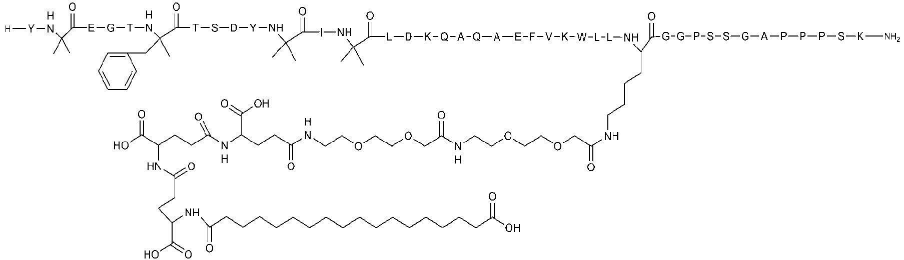
Compound P019: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA-γGlu-CO-(CH₂)₁₆-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO: 10) 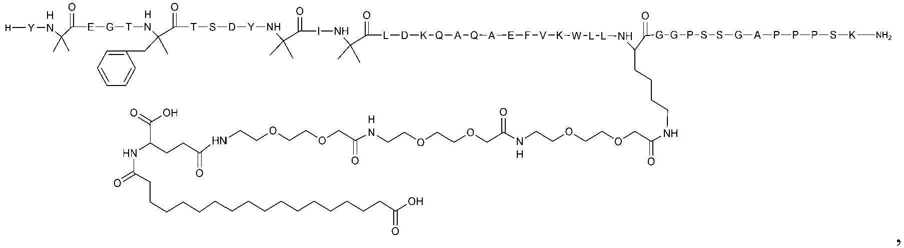 and
Compound P020: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴-Trp²⁵-Leu²⁶-Leu²⁷-Lys²⁸(AEEA-AEEA-AEEA-γGlu-CO-(CH₂)₂₀-COOH)-Gly²⁹-Gly³⁰-Pro³¹-Ser³²-Ser³³-Gly³⁴-Ala³⁵-Pro³⁶-Pro³⁷-Pro³⁸-Ser³⁹-Lys⁴⁰-NH₂ (SEQ ID NO: 11) 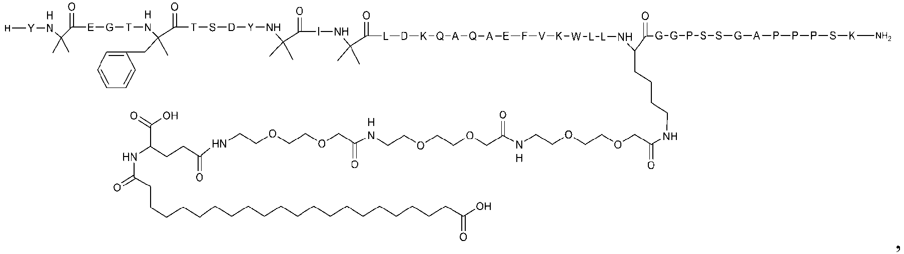
wherein AEEA represents [2-(2-amino-ethoxy)-ethoxy]-acetyl.

11. The method according to claim 9 or 10, **characterized in that** the polypeptide compound is:
Compound P016: H-Tyr¹-Aib²-Glu³-Gly⁴-Thr⁵-αMePhe⁶-Thr⁷-Ser⁸-Asp⁹-Tyr¹⁰-Aib¹¹-Ile¹²-Aib¹³-Leu¹⁴-Asp¹⁵-Lys¹⁶-Gln¹⁷-Ala¹⁸-Gln¹⁹-Ala²⁰-Glu²¹-Phe²²-Val²³-Lys²⁴(AEEA-AEEA-AEEA-γGlu-CO-(CH₂)₂₀-COOH)-Trp²⁵-Leu²⁶-Leu²⁷-Lys²⁸-Gly²⁹-Gly³⁰-Pro³¹-Ser³²-Ser³³-Gly³⁴-Ala³⁵-Pro³⁶-Pro³⁷-Pro³⁸⁻Ser³⁹-Lys⁴⁰-NH₂ (SEQ ID NO:7)

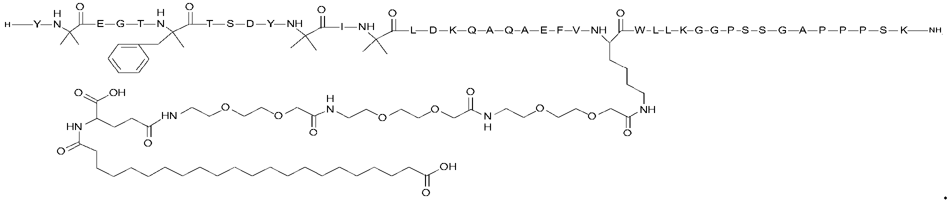

12. The method according to any one of claims 9 to 11, **characterized in that** the free α-amino group of the dipeptide fragment has an Fmoc protecting group, or a Boc protecting group, or both an Fmoc protecting group and a Boc protecting group.

13. The method according to claim 12, **characterized in that** the dipeptide fragment having an Fmoc protecting group is selected from: Fmoc-Gly-Gly-OH, Fmoc-Ile-Aib-OH, Fmoc-Tyr(tBu)-Aib-OH, Fmoc-Thr(tBu)-αMePhe-OH and Fmoc-Glu(OtBu)-Gly-OH.

14. The method according to claim 12, **characterized in that** the dipeptide fragment having a Boc protecting group is selected from: Boc-Gly-Gly-OH, Boc-Ile-Aib-OH, Boc-Tyr(tBu)-Aib-OH, Boc-Thr(tBu)-αMePhe-OH and Boc-Glu(OtBu)-Gly-OH, preferably Boc-Tyr(tBu)-Aib-OH.

15. The method according to claim 12, **characterized in that** the dipeptide fragment having both an Fmoc protecting group and a Boc protecting group is selected from: Fmoc-Gly-Gly-OH, Fmoc-Ile-Aib-OH, Fmoc-Tyr(tBu)-Aib-OH, Fmoc-Thr(tBu)-αMePhe-OH, Fmoc-Glu(OtBu)-Gly-OH and Boc-Tyr(tBu)-Aib-OH;
preferably, the dipeptide fragment having both an Fmoc protecting group and a Boc protecting group is a combination of Fmoc-Gly-Gly-OH, Fmoc-Ile-Aib-OH, Fmoc-Tyr(tBu)-Aib-OH, Fmoc-Thr(tBu)-αMePhe-OH, Fmoc-Glu(OtBu)-Gly-OH and Boc-Tyr(tBu)-Aib-OH.

16. The method according to any one of claims 9 to 15, **characterized in that** the polypeptide compound of Formula AI is divided into the following 6 fragments: Tyr¹-Aib², Glu³-Gly⁴, Thr⁵-αMePhe⁶, Tyr¹⁰-Aib¹¹, Ile¹²-Aib¹³, and Gly²⁹-Gly³⁰, as well as single amino acids at the remaining 28 sites for coupling.

17. The method according to any one of claims 9 to 16, comprising the steps of
preparing the peptide chain of the polypeptide compound of Formula AI by the solid-phase synthesis method via dipeptide fragments, and
optionally, at any lysine at the position selected from position 16, position 24, position 28 and position 40, the side chain ε-amino group is chemically modified with ([2-(2-amino-ethoxy)-ethoxy]-acetyl)a-(γ-Glu)b-CO-(CH₂)c-COOH, wherein each a is independently an integer from 0 to 5, each b is independently an integer from 0 to 5, and each c is independently an integer from 10 to 24.

18. The method according to claim 17, comprising the steps of:
preparing the peptide chain of the polypeptide compound of Formula AI by the solid-phase synthesis method via dipeptide fragments, wherein any lysine at the position selected from position 16, position 24, position 28 and position 40 has Mtt, Alloc or Dde as the protecting group for the side chain ε-amino group, and the remaining three lysines have Bocs as the protecting groups for the side chain ε-amino groups;
deprotecting the lysine having Mtt, Alloc or Dde as the protecting group for the side chain ε-amino group; and
at the side chain ε-amino group of the deprotected lysine, chemically modifying the side chain ε-amino group with ([2-(2-amino-ethoxy)-ethoxy]-acetyl)a-(γ-Glu)b-CO-(CH₂)c-COOH to obtain the polypeptide compound of Formula AI.

19. The method according to claim 18, comprising the steps of:
preparing the peptide chain of the polypeptide compound of Formula AI by the solid-phase synthesis method via dipeptide fragments; wherein the lysine at position 24 has an Mtt as the protecting group for the side chain ε-amino group, and the lysines at position 16, position 28 and position 40 have Bocs as the protecting groups for the side chain ε-amino groups;
deprotecting the side chain ε-amino group of the lysine at positions 24; and
at the deprotected side chain ε-amino group of the lysine at position 24, chemically modifying the side chain ε-amino group of the lysine at positions 24 with ([2-(2-amino-ethoxy)-ethoxy]-acetyl)a-(γ-Glu)b-CO-(CH₂)c-COOH by coupling a 2-(2-amino-ethoxy)-ethoxy]-acetyl) via Fmoc-AEEA-OH, coupling a γ-Glu via Fmoc-Glu(α-OtBu)-OH, and coupling a -OC-(CH₂)c-COO-tBu via HOOC-(CH₂)c-COOtBu;
and
performing a post-treatment, thereby obtaining the polypeptide Compound P016.
